# EUROPEAN PATENT APPLICATION

(11) **EP 2 740 804 A1**
(43) Date of publication of application: **11.06.2014**
(21) Application number: 12822928.3
(22) Date of filing: 27.07.2012
(51) Int. Cl.: C12Q 1/68, C12N 15/09

(54) **NUCLEIC ACID ANALYSIS METHOD**

(30) Priority: 05.08.2011 JP 2011172378
(71) Applicant: Kabushiki Kaisha Toshiba, Minato-ku Tokyo 105-8001 (JP)
(72) Inventor: NAKAMURA, Naoko, Tokyo 105-8001 (JP); HASHIMOTO, Koji, Tokyo 105-8001 (JP); HASHIMOTO, Michie, Tokyo 105-8001 (JP); GEMMA, Nobuhiro, Tokyo 105-8001 (JP); NIKAIDO, Masaru, Tokyo 105-8001 (JP)
(74) Representative: Arends, William Gerrit
(86) International application number: PCT/JP2012/069170
(87) International publication number: WO 2013/021841

(57) **Abstract**

Embodiments relate to a method of analyzing nucleic acid. The nucleic acid analysis method includes subjecting a sample to an amplifying reaction using the first primer and the second primer, reacting an amplification product with a nucleic acid probe, measuring presence/absence of a hybridization and/or a quantity thereof, and determining presence/absence of a target nucleic acid in the sample and/or a quantity of target nucleic acid present. The amplification product obtained with the first primer and/or second primer forms a first stem-and-loop structural body. With the detection of the presence/absence of the hybridization between a nucleic acid probe complementary to a sequence of the loop structure and the amplification product, and/or the amount thereof present, the nucleic acid analysis is carried out on the sample.

## Description

### Technical Field

Embodiments described herein relate to a method of analyzing nucleic acid.

### Background Art

In gene analysis, there is one general method for detecting or quantifying a nucleic acid comprising a specific sequence, by using a PCR amplification technique. In this method, a sample is PCR-amplified, and thereafter the amplification product is analyzed as to the presence or absence of a specific sequence or the quantity of the sequence is determined.

In the PCR amplification technique, a primer set configured to amplify the aimed sequence to be detected or quantitatively determined is used, and the amplification is carried out using a nucleic acid contained in the sample as a template nucleic acid. The thus amplification product obtained is decomposed or separated into a single strand nucleic acid by a thermal treatment or the like, and then reacted with a nucleic acid probe configured to detect the aimed sequence, for example. Based on the presence or absence or the quantity of hybridization caused thereby as an index, the aimed sequence is finally detected and/or quantitatively determined.

Depending on its sequence, a single strand obtained from an amplification product has a tendency to form a secondary structure. An amplification product obtained by the PCR amplification method comprises double strands complementary to each other. Therefore, each sample is rendered single-stranded in order to be subjected to the detection and/or quantitative determination, but the single-stranded samples are of two types of single strands comprising sequences complementary to each other. As a result, there is a tendency that complementary strands encounter with each other in a sample to spontaneously form double strands. These tendencies of the single strand may adversely affect the performance of the detection and/or quantitative determination.

### Disclosure of Embodiments

### [Summary of the Embodiments]

### [Object to be Achieved by the Embodiments]

An object to be achieved by the embodiments is to provide a nucleic acid analysis method which can be performed precisely and easily in a short time.

### [Means for Achieving the Object]

The present embodiments relate to a nucleic acid analysis method for a sample. The nucleic acid analysis method comprises amplifying a sample with a first primer and a second primer; reacting an amplification reaction product with a nucleic acid probe; detecting presence or absence of hybridization and/or a quantity thereof; and determining presence or absence of a target nucleic acid and/or a quantity of the target nucleic acid in the sample. The amplification product by the first primer and/or second primer forms a stem-and-loop structure. By detecting the presence or absence of hybridization between a nucleic acid probe complementary to the sequence of the loop structure and the amplification product, and/or the quantity of the hybridization, the nucleic acid analysis of the sample is carried out.

### Brief Description of Drawings

FIG. 1 is a schematic diagram showing an example of the amplification process according to an embodiment;
FIG. 2 is a schematic diagram showing an example of the amplification process according to an embodiment;
FIG. 3 is a schematic diagram showing an example of the amplification process according to an embodiment;
FIG. 4 is a schematic diagram showing an example of the amplification process according to an embodiment;
FIG. 5 is a schematic diagram showing an example of the amplification process according to an embodiment;
FIG. 6 is a schematic diagram showing an example of the amplification process according to an embodiment;
FIG. 7 is a diagram showing designed positions for primers and probes of Example 1;
FIG. 8 is a diagram showing the result of electrophoresis in Example 1;
FIG. 9 is a graph showing the result of detection in Example 1;
FIG. 10 is a graph showing the result of detection in Example 2;
FIG. 11 is a graph showing the result of detection in Example 2;
FIG. 12 is a graph showing the result of detection in Example 3;
FIG. 13 is a diagram showing an example of a DNA chip in an embodiment; and
FIG. 14 is a diagram showing an example of a DNA chip in an embodiment.

### Best Mode for Carrying Out the Invention

The present embodiments will now be described in detail.

### [Definitions]

The term "amplification" or "amplification reaction" is defined as the repeated replication a template nucleic acid using a primer set, and comprises such processing steps as elongation and amplification. The amplification may be any method conventionally known by itself, for replicating a template nucleic acid basically with two primers, for example, a set of a forward primer and a reverse primer. Preferable examples of this method are a PCR amplification, LCR amplification, RCA amplification, TMA amplification, NASBA amplification, SDA amplification and ICAN amplification. Further, if necessary, a reverse transcription reaction, which uses a reverse transcriptase, may be used at the same time in combination with the amplification reaction. Any conventionally known technique may be used when carrying out the amplification and reverse transcription at the same time.

"Nucleic acid" is a term which collectively covers substances whose structure, in part, can be expressed by a nucleotide sequence, such as DNA, RNA, PNA, LNA, S-oligo and methylphosphonateoligo. For example, a nucleic acid may be DNA or RNA of nature origin, or a partially or thoroughly artificially synthesized and/or designed nucleic acid or a mixture of any of these.

The term "sample" is defined as an object to be subjected to the analysis method according to this embodiment, and it can only be a specimen which may contain nucleic acid. It is preferred here that the sample be in such a state that it does not hinder the amplification and/or hybridization reaction. For example, when using a material obtained from a living organism or the like as a sample of this embodiment, it may be subjected to a pretreatment using any means conventionally known by itself. Further, the sample may be in the form of liquid, for example.

Nucleic acids contained in samples are called "sample nucleic acids". Of the sample nucleic acids, those containing polynucleotide to be replicated by annealing the primer are called "templates". In the templates, the portion of a template which is defined by two primers contained in a primer set is called "template nucleic acid". Further, a region of a "template nucleic acid", excluding a region to which the primer binds (also called an "annealing region"), is called, here, a "template sequence".

The term "target sequence" is defined as a sequence to be detected or quantitatively determined using the nucleic acid probe.

The term "target nucleic acid" is defined as a nucleic acid containing a target sequence, and specifically, it refers to as, of those replicated with the primer from a template nucleic acid, a nucleic acid containing a target sequence. When replication with the primer is elongation, the replicate is an elongation product containing a target sequence, and when replication with the primer is amplification, the replicate is referred to as an "amplification product" containing a target sequence. The "elongation product" or "amplification product" is also called as "replication product".

The term "nucleic acid probe" is defined as a nucleic acid containing a sequence complementary to a target sequence. A nucleic acid probe may be used in a released state, or immobilized to a solid phase conventionally known by itself. A preferable nucleic acid probe is immobilized to a surface of a substrate when used. A device which comprises such a substrate and a nucleic acid probe immobilized on the substrate may be realized generally by a DNA chip.

The "DNA chip" is a device configured to analyze nucleic acids by utilizing the hybridization reaction between a nucleic acid probe containing a sequence complementary to a nucleic acid to be detected and the object nucleic acid to be detected. The term "DNA chip" is synonymous with "nucleic acid chip", "microarray", "DNA array" or the like, which may be generally used, and these terms can be used interchangeably.

The term "sequence originated from a template sequence" (or "sequence originated from a sequence complementary to a template sequence") is defined as a sequence which reflects sequence information on a sequence of a region contained in a template sequence (or a complementary sequence thereof) defined by a set of primers after each primer annealing to the template sequence (or the complementary sequence thereof), that is, the information on the nucleotide sequence, the direction of the sequence or the like.

The terms "complementary" and "complementary strand" may be used when two single-strand nucleic acids have complementation with each other of at least 80%, at least 85%, at least 90%, at least 95% or 100%. The terms "homologous" and "homologous strand" may be used when two single-strand nucleic acids have homology with each other of at least 80%, at least 85%, at least 90%, at least 95% or 100%.

### (1) Template Nucleic Acid

First, a sample is prepared before carrying out a nucleic acid analysis method according to an embodiment. The sample may be any object as long as it possibly contains a nucleic acid and is supposed to be analyzed with regard to the nucleic acid contained therein. It is necessary in the sample that the amplification reaction be carried out appropriately, and therefore a crude sample obtained from an object to be analyzed may be subjected to a pre-treatment to prepare the sample. Alternatively, when the object is appropriate for the amplification reaction, the crude sample itself may be used without being subjected to a pre-treatment as a sample as it is.

When there are nucleic acids in a sample, which have a sequence to be analyzed, these nucleic acids are used as the first template nucleic acids. The sample nucleic acid to be used as a template nucleic acid may be single-strand or double-strand. Even when amplification is started for a single-strand sample nucleic acid, a complementary strand thereof is replicated in the amplification process. Therefore, the template nucleic acid which exists from the beginning functions as the first template nucleic acid, whereas the complementary strand of the template nucleic acid, which is replicated and formed in the amplification process, functions as the second template nucleic acid.

### (2) Stem-and-loop structural body

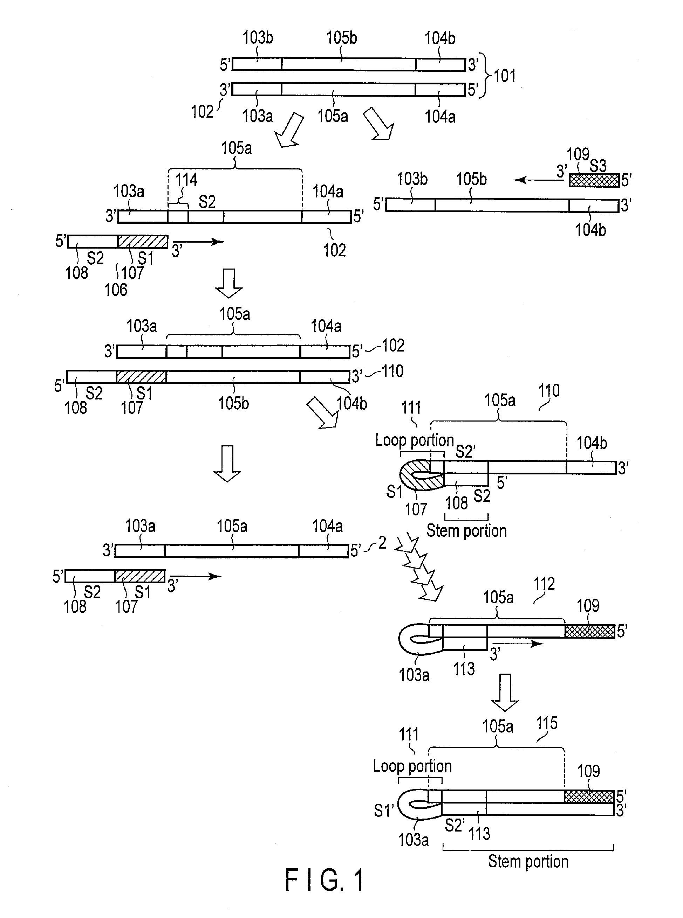

The stem-and-loop structural body has an intramolecular structure indicated by reference numeral 110 in FIG. 1, for example, and comprises a single-strand loop portion and a double-strand portion, the double-strand portion including a stem portion.

In this embodiment, two stem-and-loop structural bodies are formed. One is a stem-and-loop structural body indicated by the reference numeral 110, which comprises a stem portion located on a 5' terminal side, a loop portion located adjacent to a 3' side of the stem portion, a double strand portion comprising a sequence complementary to the stem portion and located on a 3' side of the loop portion, and a straight chain single-strand portion (see FIG. 1). Whereas the other one is a stem-and-loop structural body indicated by the reference numeral 115. This stem-and-loop structural body comprises a single-strand region comprising a loop portion and a double-strand region containing a stem portion. These two types of stem-and-loop structural bodies can be obtained by inducing an amplification reaction using a primer set of a forward primer and a reverse primer to one template nucleic acid.

### (3) Nucleic acid analysis method

The nucleic acid analysis method, which is an example of the embodiment, will now be described.

First, a sample to be analyzed is amplified with the forward primer and reverse primer. Next, the amplification product thus obtained is reacted with a nucleic acid probe containing a sequence complementary to a specific target sequence. Further, hybridization between the nucleic acid probe and the amplification product is detected and/or the hybridization amount is measured. Based on this result, sequence data on the sample nucleic acid contained in the sample can be obtained.

Here, the items to be analyzed are, for example, the measurement of the amount of gene expression, the quantitative determination of a nucleic acid or the like originated from virus and/or bacteria or the like, the identification of the genotype of polymorphism, and/or the detection of the presence or absence of a mutation. But, the items of the embodiment are not limited to those mentioned above.

### <Amplification Process>

In the amplification process, an indicator nucleic acid which reflects information to be obtained in this analysis is selected, and based on this, the template nucleic acid is designed.

A double-strand nucleic acid 101 to be amplified contains a first template nucleic acid 102 and a second template nucleic acid, a complementary strand thereof. The first template nucleic acid 102 comprises a first sequence 103a located on a 3' terminal, a first template sequence 105a adjacent to a 5' side of the first sequence, and a second sequence 104a adjacent to a 5' side of the first template sequence and located on the 5' terminal. The second template nucleic acid comprises a third sequence 104b located on the 3' terminal, a second template sequence 105b adjacent to a 5' side of the third sequence, and a fourth sequence 103b adjacent to a 5' side of the second template sequence and located on the 5' terminal.

For the amplification of these template nucleic acids, the forward primer and reverse primer are selected as required. FIG. 1 shows a first primer 106 and a second primer 109. Either one of the first primer 106 and second primer 109 may be a forward primer or reverse primer or vice versa. In any case, a similar amplification product can be obtained by a similar procedure.

The first primer 106 contains an S1 sequence complementary to the first sequence 103a of the first template nucleic acid 102 and an S2 sequence 108 adjacent and joined to a 5' side of the S1 and located on the 5' terminal. In the amplification reaction, the first primer 106 is bound to the first sequence 103a and elongated, and thus an elongated strand 101 is formed. Here, the S2 sequence 108 is designed to contain a sequence complementary to a part of the template sequence 105a contained in the first template nucleic acid 102.

On the other hand, the second primer 109 is bound to the third sequence 104b of the second template nucleic acid in the amplification reaction, and then the second primer 109 is elongated, and thus an elongated strand (not shown) is formed.

The elongated strand 110 of the first primer 106 is separated by denaturation into two single-strand replication strands to form the first replication strand 110 which corresponds to the elongated strand 110 of the first primer 106. The first replication strand 110 forms an intramolecular structure spontaneously in the reaction liquid. The intramolecular structure is a stem-and-loop structure. The first replication strand 110 forms a double-strand region between a stem portion 108 and a S2' sequence, the stem portion 108 corresponding to the S2 sequence derived from the first primer, and the S2' sequence being a complementary sequence of the S2 sequence and being a part of the complementary sequence 105b of the first template sequence. Upon formation of this double-strand region, a single-strand loop portion 111 is formed. The loop portion 111 may include the S1 sequence derived from the first primer and a part of the complementary sequence 105b of the first template sequence.

The length of the sequence contained in the loop portion 111 and derived from the complementary sequence 105b of the first template sequence is determined by selecting the sequence for the S2 sequence of the first primer. In other words, the length of the sequence derived from the template sequence contained in the loop portion of the stem-and-loop structural body to be finally obtained is determined based on how remote the position of the sequence is from the 5' terminal of the first sequence 103a towards the 3' direction, for which the S2 sequence is complementary. As to the sequence of the loop portion, the target sequence to be detected with the nucleic acid probe is designed as will be described later. When a sequence derived from the template sequence is contained in the target sequence, such a detection result as a false positive, which may be caused by detecting a detection signal derived from an unreacted primer, can be effectively excluded.

In other words, it is possible to select the length of the sequence originated from the template sequence contained in the loop portion by determining the length of a recognition sequence 114 located on a further 5' side than the 5' terminal of the first sequence 103a and present on the 3' side of the 3' terminal of the S2' sequence indicated by "S2'". The recognition sequence 114 may be detected as a target sequence with the nucleic acid probe together with a sequence of at least a part of the S1 sequence. Therefore, it is further advantageous if the length of the sequence is recognizable as a sequence derived from the template sequence.

As described above, the amplification process according to one embodiment is explained by taking an example in which a recognition sequence, which is a sequence derived from the template sequence, is contained in the loop portion of the stem-and-loop structural body, which is a replication strand, and further the recognition sequence is contained in the target sequence to be detected with the nucleic acid probe. But, the recognition sequence may not necessarily be contained in the loop portion. That is, the primer set may be designed such that a recognition sequence is not present or the loop portion does not contain a sequence derived from the recognition sequence. For example, with utilization of such a detection principle that can detect double strands including a stem sequence, a detection signal derived from an unreacted primer and a signal derived from an amplification product can be detected while distinguishing these signals from each other by utilizing the presence of the double-strand sequence. For this reason, a recognition sequence is not necessarily required. Note that in this case, it is advantageous if the double-strand portion including the stem sequence has a certain length as compared to the case where the portion is short.

For example, the recognition sequence 114 may be of 0 base or more, 1 base or more, 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 13 bases or more, 14 bases or more, 15 bases or more, 20 bases or more, 25 bases or more, 30 bases or more or 35 bases or more, and also 50 bases or fewer, 40 bases or fewer, 35 bases or fewer, 30 bases or fewer, or 25 bases or fewer or 20 bases or fewer, or may be that in a range of combination of any of these lower limit numbers and higher limit numbers. Preferably, it should be of 20 to 80 bases, and more preferably, 30 to 50 bases. Further, the length may be selected in consideration of the length of the template nucleic acid to be analyzed.

The length of the template nucleic acid may be selected appropriately according to, for example, the template nucleic acid to be analyzed, the sequence to be analyzed and the detection principle in the detection step, which will be later explained. For example, it may be of 15 bases or more, 20 bases or more, 25 bases or more, 30 bases or more, 35 bases or more, 40 bases or more, 45 bases or more, 50 bases or more, 60 bases or more, 70 bases or more, 80 bases or more, 90 bases or more, 100 bases or more, 150 bases or more, 200 bases or more, 250 bases or more, 300 bases or more, 400 bases or more, 500 bases or more, 600 bases or more, 800 bases or more, 900 bases or more, or 1000 bases or more, and also 1500 bases or fewer, 1000 bases or fewer, 900 bases or fewer, 800 bases or fewer, 700 bases or fewer, 600 bases or fewer, 500 bases or fewer, 400 bases or fewer, 300 bases or fewer, 200 bases or fewer, 150 bases or fewer, 100 bases or fewer, 90 bases or fewer, 80 bases or fewer, 70 bases or fewer, 65 bases or fewer, 60 bases or fewer, 55 bases or fewer, 50 bases or fewer, 45 bases or fewer, 40 bases or fewer, 35 bases or fewer, 30 bases or fewer, 25 bases or fewer, 20 bases or fewer or 15 bases or fewer, or may be that in a range of combination of any of these lower limit numbers and higher limit numbers. Preferably, it should be of 50 to 1000 bases, and more preferably, 100 to 300 bases.

The length of the first primer may be selected appropriately according to, for example, the template nucleic acid to be analyzed, the sequence to be analyzed and the detection principle in the detection step, which will be later explained. For example, the length of the first primer may be 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 13 bases or more, 14 bases or more, 15 bases or more, 20 bases or more, 25 bases or more, 30 bases or more, 35 bases or more, 40 bases or more, 45 bases or more, 50 bases or more, 60 bases or more, 70 bases or more, 80 bases or more, 90 bases or more, 100 bases or more, 110 bases or more, 120 bases or more, 150 bases or more or 200 bases or more, and also 200 bases or fewer, 150 bases or fewer, 130 bases or fewer, 120 bases or fewer, 110 bases or fewer, 100 bases or fewer, 90 bases or fewer, 80 bases or fewer, 70 bases or fewer, 60 bases or fewer, 50 bases or fewer, 40 bases or fewer, 35 bases or fewer, 30 bases or fewer, 25 bases or fewer, 20 bases or fewer, or may be that in a range of combination of any of these lower limit numbers and higher limit numbers. Preferably, it should be of 20 to 100 bases, and more preferably, 30 to 60 bases. Alternatively, the length of the first primer may be, for example, 13 to 40 bases, 15 to 30 bases, 16 to 47 bases or 18 to 37 bases.

The length of each of the S1 sequence and S2 sequence may be 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 13 bases or more, 14 bases or more, 15 bases or more, 20 bases or more, 25 bases or more, 30 bases or more, 35 bases or more, 40 bases or more, 45 bases or more, 50 bases or more, 60 bases or more, 70 bases or more, and also 80 bases or fewer, 75 bases or fewer, 70 bases or fewer, 65 bases or fewer, 60 bases or fewer, 55 bases or fewer, 50 bases or fewer, 45 bases or fewer, 40 bases or fewer, 35 bases or fewer, 30 bases or fewer, 25 bases or fewer or 20 bases or fewer, or may be that in a range of combination of any of these lower limit numbers and higher limit numbers. Preferably, it should be of 10 to 50 bases, and more preferably, 15 to 30 bases.

The length of the second primer may be, for example, 2 bases or more, 3 bases or more, 4 bases or more, 5 bases or more, 6 bases or more, 7 bases or more, 8 bases or more, 9 bases or more, 10 bases or more, 11 bases or more, 12 bases or more, 13 bases or more, 13 bases or more, 14 bases or more, 15 bases or more, 20 bases or more, 25 bases or more, 30 bases or more, 35 bases or more, 40 bases or more, 45 bases or more, 50 bases or more, 55 bases or more, 60 bases or more, 65 bases or more, 70 bases or more, 80 bases or more, 90 bases or more, 100 bases or more or 110 bases, and also 150 bases or fewer, 120 bases or fewer, 110 bases or fewer, 100 bases or fewer, 90 bases or fewer, 80 bases or fewer, 70 bases or fewer, 60 bases or fewer, 50 bases or fewer, 40 bases or fewer, 35 bases or fewer, 30 bases or fewer, 25 bases or fewer, 20 bases or fewer, 15 bases or fewer, 10 bases or fewer, or may be that in a range of combination of any of these lower limit numbers and higher limit numbers. Preferably, it should be of 10 to 100 bases, and more preferably, 15 to 60 bases. Alternatively, the length of the second primer may be, for example, 13 to 40 bases, 15 to 30 bases, 16 to 47 bases or 18 to 37 bases.

The length of the replication strand having the stem-and-loop structure may be selected appropriately according to, for example, the template nucleic acid to be analyzed, the sequence to be analyzed and the detection principle in the detection step, which will be later explained. Although it is not limited to the following, the length may be, for example, about 5 bases or more, about 10 bases or more, about 15 bases or more, about 20 bases or more, about 30 bases or more, about 35 bases or more, about 40 bases or more, about 45 bases or more, about 50 bases or more, about 55 bases or more, about 60 bases or more or about 65 bases or more, and also about 100 bases or fewer, about 95 bases or fewer, about 90 bases or fewer, about 85 bases or fewer, about 80 bases or fewer, about 75 bases or fewer, about 70 bases or fewer, about 65 bases or fewer, about 60 bases or fewer, about 55 bases or fewer, about 50 bases or fewer, about 45 bases or fewer, about 40 bases or fewer, about 35 bases or fewer, about 30 bases or fewer, about 25 bases or fewer or about 20 bases or fewer, or may be that in a range of combination of any of these lower limit numbers and higher limit numbers. For example, it may be about 10 to about 80 bases, about 10 to about 60 bases, about 20 to about 80 bases or about 20 to about 60 bases. For example, it suffices if the replication strand is designed to be able to obtain a desired intra-molecular structure.

The length of the double strand including the stem portion in the stem-and-loop structural body may be selected appropriately according to, for example, the template nucleic acid to be analyzed, the sequence to be analyzed and the detection principle in the detection step, which will be later explained. Although it is not limited to the following, the length may be, for example, about 5 bases or more, about 10 bases or more, about 15 bases or more, about 20 bases or more, about 30 bases or more, about 35 bases or more, about 40 bases or more, about 45 bases or more, about 50 bases or more, about 55 bases or more, about 60 bases or more or about 65 bases or more, about 70 bases or more, about 75 bases or more, about 80 bases or more, about 90 bases or more, about 100 bases or more, about 110 bases or more, about 120 bases or more, about 130 bases or more, about 140 bases or more, about 150 bases or more, about 200 bases or more, about 300 bases or more, about 500 bases or more, about 800 bases or more or about 1000 bases or more, and also about 1800 bases or fewer, about 1500 bases or fewer, about 1300 bases or fewer, about 1200 bases or fewer, about 1100 bases or fewer, about 1000 bases or fewer, about 900 bases or fewer, about 800 bases or fewer, about 700 bases or fewer, about 600 bases or fewer, about 500 bases or fewer, about 400 bases or fewer, about 350 bases or fewer, about 300 bases or fewer, about 250 bases or fewer, about 200 bases or fewer, about 150 bases or fewer, about 100 bases or fewer, about 95 bases or fewer, about 90 bases or fewer, about 85 bases or fewer, about 80 bases or fewer, about 75 bases or fewer, about 70 bases or fewer, about 65 bases or fewer, about 60 bases or fewer, about 55 bases or fewer, about 50 bases or fewer, about 45 bases or fewer, about 40 bases or fewer, about 35 bases or fewer, about 30 bases or fewer, about 25 bases or fewer or about 20 bases or fewer, or may be that in a range of combination of any of these lower limit numbers and higher limit numbers. Preferably, it should be 10 to 1000 bases, or more preferably 15 to 300 bases.

The length of the single strand in the stem-and-loop structural body may be selected appropriately according to, for example, the template nucleic acid to be analyzed, the sequence to be analyzed and the detection principle in the detection step, which will be later explained. Although it is not limited to the following, the length may be, for example, about 3 bases or more, about 4 bases or more, about 5 bases or more, about 6 bases or more, about 7 bases or more, about 8 bases or more, about 9 bases or more, about 10 bases or more, about 11 bases or more, about 12 bases or more, about 13 bases or more, about 14 bases or more, about 15 bases or more, about 20 bases or more, about 30 bases or more, about 35 bases or more, about 40 bases or more, about 45 bases or more, about 50 bases or more, about 55 bases or more, about 60 bases or more or about 65 bases or more, about 70 bases or more, about 80 bases or more, about 90 bases or more, about 100 bases or more, about 110 bases or more, about 120 bases or more, about 130 bases or more, about 140 bases or more, about 150 bases or more, about 160 bases or more, about 170 bases or more, about 180 bases or more, about 190 bases or more, about 200 bases or more or about 250 bases or more, and also about 300 bases or fewer, about 250 bases or fewer, about 200 bases or fewer, about 190 bases or fewer, about 180 bases or fewer, about 170 bases or fewer, about 160 bases or fewer, about 150 bases or fewer, about 140 bases or fewer, about 130 bases or fewer, about 120 bases or fewer, about 110 bases or fewer, about 100 bases or fewer, about 95 bases or fewer, about 90 bases or fewer, about 85 bases or fewer, about 80 bases or fewer, about 75 bases or fewer, about 70 bases or fewer, about 65 bases or fewer, about 60 bases or fewer, about 55 bases or fewer, about 50 bases or fewer, about 45 bases or fewer, about 40 bases or fewer, about 35 bases or fewer, about 30 bases or fewer, about 25 bases or fewer, about 20 bases or fewer, about 15 bases or fewer, about 10 bases or fewer or about 8 bases or fewer, or may be that in a range of combination of any of these lower limit numbers and higher limit numbers. Preferably, it should be 10 to 200 bases, or more preferably 15 to 60 bases.

Further, in the amplification reaction, a further replication strand can be obtained by repeating the amplification process using the replication strand obtained in the amplification step as the template nucleic acid. That is, a replication strand 112 illustrated in FIG. 1, the right hand side thereof, the second row from the bottom, is obtained as the second replication strand corresponding to the elongated strand of the second primer, and details thereof will be explained later. The replication strand 112 comprises an S2' sequence 113 complementary to an S2 sequence located on the 3' terminal, an S1' sequence 103a complementary to an S1 sequence adjacent thereto on the 5' side, a complementary sequence 105a of the first template sequence adjacent thereto on the 5' side, and a second primer sequence 109 adjacent thereto on the 5' side. The second replication strand forms a stem-and-loop structure as in the case of the first replication strand 110. More specifically, the S2' sequence 113 is bound to the sequence which is a part of the complementary sequence 105a of the first template sequence, which is complementary to the S2' sequence 113, and thus the loop portion 114 is formed. Further, particularly, in the second replication strand, the 3' terminal is further elongated by polymerase existing in the reaction field. Accordingly, the stem portion 113 extends along the complementary strand, and thus a double-strand containing a further elongated stem portion can be obtained.

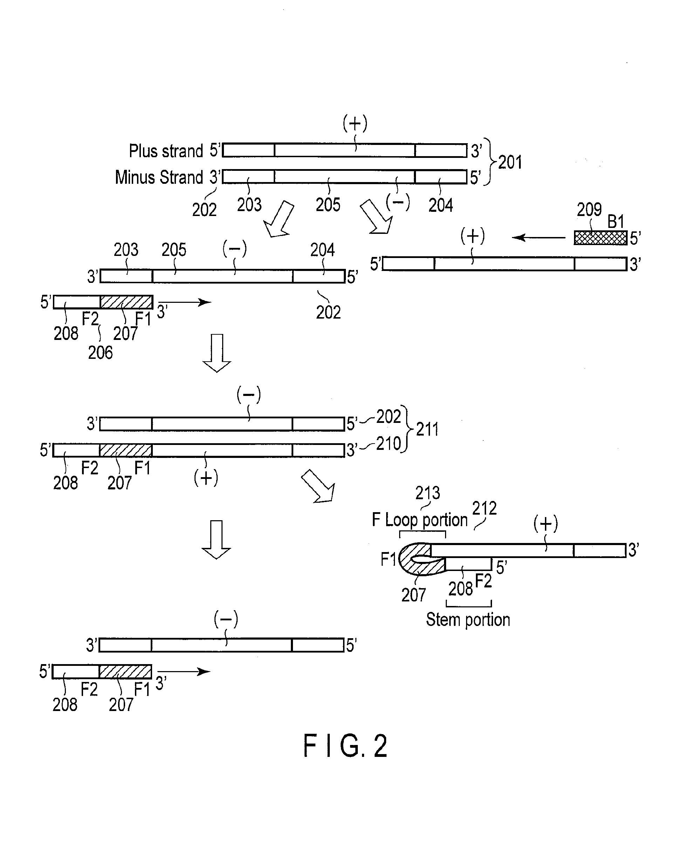

FIG. 2 shows an example of the amplification in the case where the first primer is used as a forward primer 206 and the second primer used as a reverse primer 209. This figure illustrates, in particular, a plus strand and a minus strand of the template nucleic acid for clarification. Here, the amplification reaction is started and proceeds in a similar manner to that of FIG. 1 except that the S1 sequence, S2 sequence and S3 sequence in FIG. 1 are referred to as "F1 sequence", "F2 sequence" and "B1 sequence", and the first template nucleic acid is assigned as the minus strand (sign "(-)" is indicated for the sequence in each stage) and the second template nucleic acid is assigned as the plus strand (sign "(+)" is indicated for the sequence in each stage).

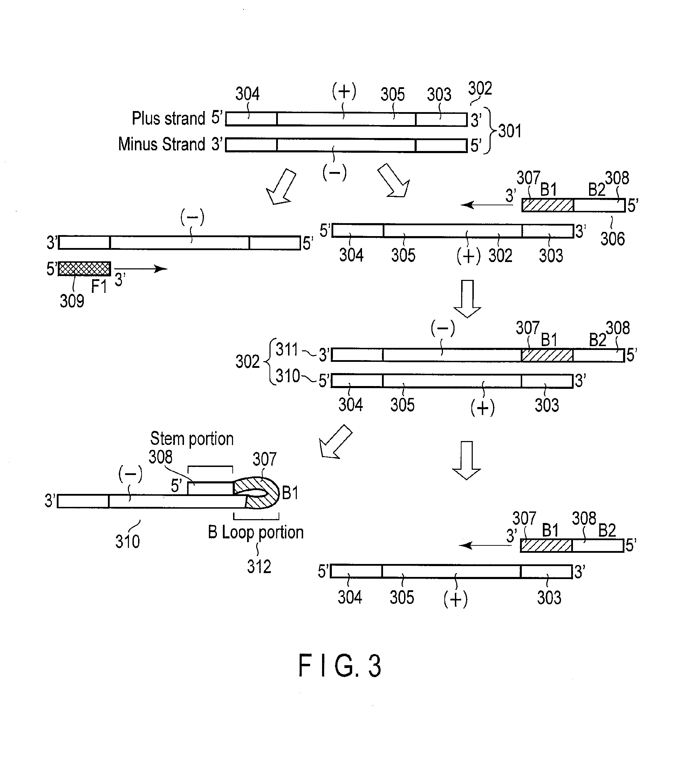

FIG. 3 illustrates the amplification in the case where the first primer is used as a reverse primer 306 and the second primer used as a forward primer 309 to clarify, in particular, the plus strand and the minus strand of the template nucleic acid. Here, the amplification reaction is started and proceeds in a similar manner to that of FIG. 1 except that the S1 sequence, S2 sequence and S3 sequence in FIG. 1 are referred to as "B1 sequence", "B2 sequence" and "F1 sequence", and the first template nucleic acid is assigned as the plus strand (sign "(+)" is indicated for the sequence in each stage) and the second template nucleic acid is assigned as the minus strand (sign "(-)" is indicated for the sequence in each stage).

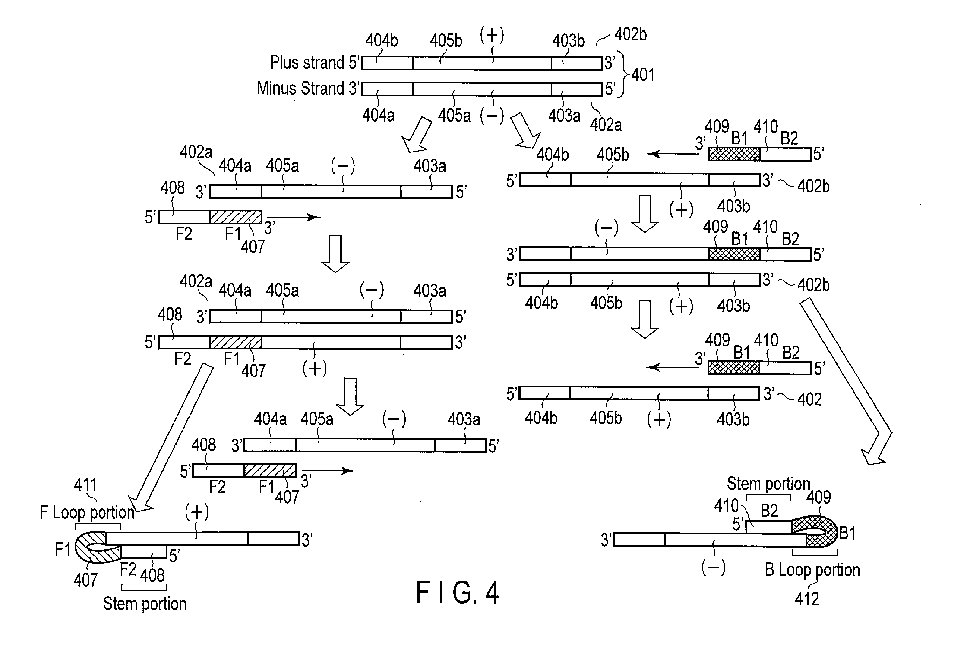

FIG. 4 shows an embodiment in which the first primer contains the S1 sequence and S2 sequence, and as well, the second primer contains, in addition to the S3 sequence, an S4 sequence on the 5' terminal side thereof. More specifically, the first primer is used as the forward primer, and contains the F1 sequence corresponding to the S1 sequence and the F2 sequence corresponding to the F2 sequence. On the other hand, the second primer is used as the reverse primer, and contains the B1 sequence corresponding to the S3 sequence and the B2 sequence corresponding to the S4 sequence.

Each of the above-described amplification steps is carried out in a similar manner to that of FIG. 1 except for the following. That is, with the amplification reaction shown in FIG. 1, two types of stem-and-loop structural bodies are obtained, whereas with the amplification reaction shown in FIG. 4, a total of four types of stem-and-loop structural bodies are obtained. Of these, two types each comprise the first replication strand corresponding to the elongated strand of the first primer and the second replication strand corresponding to the elongated strand of the second primer as in FIG. 1. The other two types each comprise the first replication strand corresponding to the elongated strand of the first primer and the second replication strand corresponding to the elongated strand of the second primer, obtained in the amplification step shown in FIG. 3.

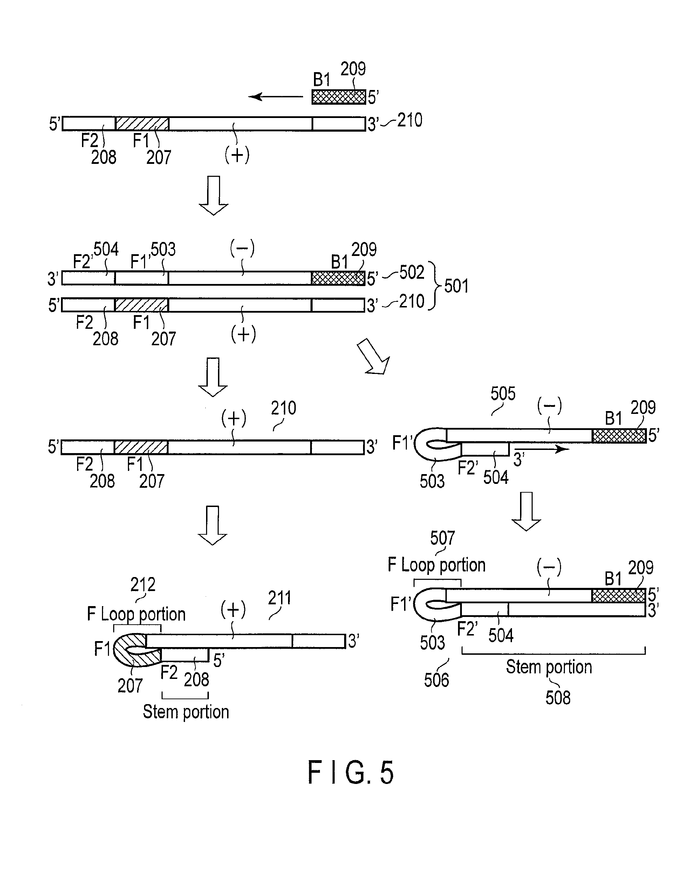

FIG. 5 shows the process of forming a replication strand corresponding to the elongated strand of the second primer shown in FIG. 1. This is shown as the subsequent amplification process following the reaction of FIG. 2, which further specifically illustrates FIG. 1. The replication strand used as the first template in FIG. 5 is a replication strand 210 corresponding to the elongated strand of the forward primer 206 in FIG. 2. In FIG. 5, the reverse primer 209 bound to the replication strand 210 is elongated to generate an elongated strand 502. The elongated strand 502 forms an intramolecular structure spontaneously while depending on the sequence thereof, and thus a stem-and-loop structural body 505 is formed. The stem-and-loop structural body 505 comprises an F2' sequence located on the 3' terminal (the sequence being complementary to the "F2 sequence"), an F1' sequence adjacent thereto on the 5' side (the sequence being complementary to the "F1 sequence"), a template sequence of a minus strand, adjacent thereto on the 5' side, and a B1 sequence adjacent thereto on the 5' side. The F2' sequence contains therein a sequence complementary to a part of the template sequence of the minus strand, and therefore, an intramolecular binding occurs on the same strand. Thus, a double strand region containing a stem portion and a single strand region containing a loop portion are formed. Further, the 3' terminal of the stem-and-loop structural body 505 is further elongated by polymerase in the reaction liquid. With this elongation, all the regions are double-stranded except for the loop portion 508.

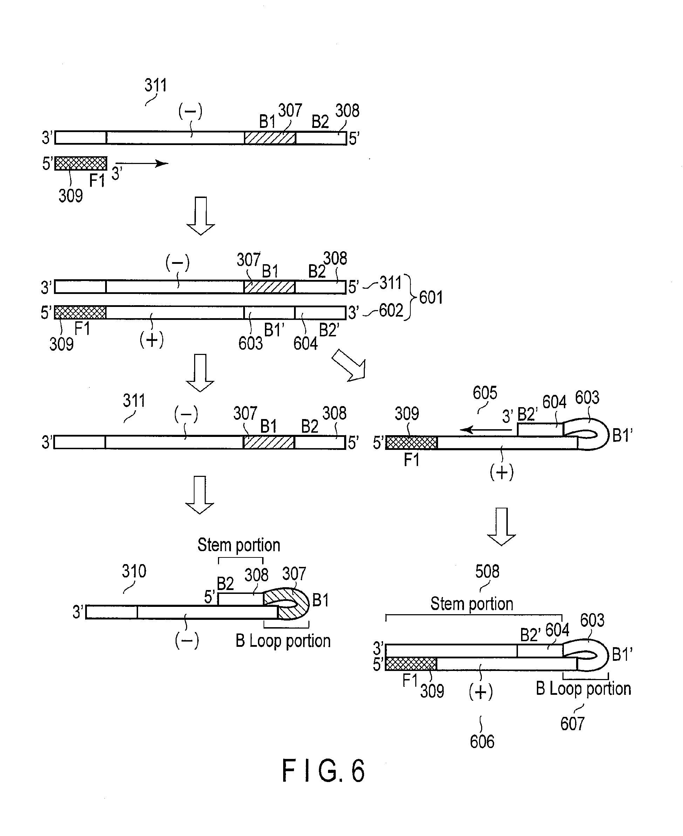

FIG. 6 shows the process of further forming a replication strand in which all the regions of the stem-and-loop structural body are double stranded except for the loop portion as in the case shown in FIG. 5. In FIG. 6, an elongated strand 311 of the reverse primer 308 shown in FIG. 3 is used as the first template nucleic acid. In FIG. 6, a forward primer 309 bound to an elongated strand 311 is elongated to generate an elongated strand 602. The elongated strand 602 forms an intramolecular structure spontaneously, and thus a stem-and-loop structural body 605 is formed. The stem-and-loop structural body 605 comprises a B2' sequence located on the 3' terminal (the sequence being complementary to the "B2 sequence"), a B1' sequence adjacent thereto on the 5' terminal side (the sequence being complementary to the "B1 sequence"), a template sequence of a plus strand, adjacent thereto on the 5' terminal side, and an F1 sequence adjacent thereto on the 5' terminal side. The B1' sequence contains therein a sequence complementary to a part of the template sequence of the plus strand, and therefore, an intramolecular binding occurs on the same strand. Thus, a double strand region containing a stem portion and a single strand region containing a loop portion are formed. Further, the 3' terminal of the stem-and-loop structural body 605 is further elongated. With this elongation, all the regions of the stem-and-loop structural body 606 are double stranded except for the loop portion 607.

### <Primer Set>

As to the primer set used in the embodiment, it may contain two types of primers which can be used in any amplification reaction conventionally known by itself, with which the amplification is achieved by two primers, that is, the first primer and the second primer.

In the case of the PCR amplification, the first primer may be a forward primer or a reverse primer. When the first primer is a forward primer, the second primer may be a reverse primer. On the other hand, when the first primer is a reverse primer, the second primer may be a forward primer.

For example, in the case where a nucleic acid to be amplified contains the first template nucleic acid and the second template nucleic acid, and the first template nucleic acid contains a first sequence located on a 3' terminal, a first template sequence adjacent to a 5' side thereof and a second sequence adjacent to a 5' side thereof and located on the 5' terminal, whereas the second template nucleic acid is a strand complementary to the first template nucleic acid, the second template nucleic acid contains a third sequence located on the 3' terminal, a second template sequence adjacent to a 5' side thereof, and a fourth sequence adjacent to a 5' side thereof and located on the 5' terminal, the first primer and the second primer may have the following structures, respectively.

### [Example 1]

The first primer contains an S1 sequence and an S2 sequence, the S1 sequence is complementary to the first sequence and the S2 sequence bound to a 5' terminal side of the S1 sequence. The S2 sequence is a sequence located adjacent to a 5' side of the S1 sequence or in the vicinity of the S1 sequence, in which The S2 sequence is at least a part of the first template nucleic acid.

The second primer contains an S3 sequence, which is complementary to the third sequence.

A part of the amplification product obtained when the template nucleic acid is amplified using such primers is a stem-and-loop structural body. This stem-and-loop structural body has the following two structures.

The first replication strand corresponding to the elongated strand of the first primer forms the first stem-and-loop structural body which comprises a first double strand region and a first single strand region on one strand, the first double strand region containing a first stem portion, and the first single strand region containing a first loop portion. The first double strand region contains the first stem portion derived from the S2 sequence and a sequence derived from the complementary sequence of the first template sequence complementary to the first stem portion. The first single strand region, which forms the first loop portion, contains an S1 sequence and, optionally, a sequence derived from the complementary sequence of the first template sequence adjacent to a 3' side thereof. Here, in regarding to the first primer, in the case where the S2 sequence is designed to be adjacent to a 5' side of the first sequence, the first single strand region, which forms the first loop portion, may not contain a sequence derived from the complementary sequence of the first template sequence adjacent to a 3' side of the S1 sequence.

The second replication strand corresponding to the elongated strand of the second primer forms the second stem-and-loop structural body which comprises a second double strand region and a second single strand region on one strand, the second double strand region containing a second stem portion, and the second single strand region containing a second loop portion. The second double strand region contains the second stem portion derived from the complementary sequence of the S2 sequence and a sequence derived from the complementary sequence of the second template sequence complementary to the second stem portion. The second single strand region, which forms the second loop portion, contains a complementary sequence of an S1 sequence and, optionally, a sequence derived from the complementary sequence of the second template sequence adjacent to a 5' side thereof. Here, in regarding to the first primer, in the case where the S2 sequence is designed to be adjacent to a 5' side of the first sequence, the second single strand region, which forms the second loop portion, may not contain a sequence derived from the complementary sequence of the second template sequence on the 5' side of the S1 sequence.

### [Example 2]

Alternatively, the first primer and the second primer may have the following structures.

The first primer contains an S1 sequence, which is complementary to the first sequence, and an S2 sequence bound to a 5' side of the S1 sequence. The S2 sequence is a sequence located adjacent to a 5' side of the first sequence or in the vicinity thereof, the S2 sequence is a same sequence of at least a part of the first template nucleic acid.

The second primer contains an S3 sequence, which is complementary to the third sequence, and an S4 sequence bound to a 5' side of the S3 sequence. The S4 sequence is a sequence located adjacent to a 5' side of the third sequence or in the vicinity thereof, the S4 sequence is a same sequence of at least a part of the second template nucleic acid.

A part of the amplification product obtained when the template nucleic acid is amplified using such primers is a stem-and-loop structural body. This stem-and-loop structural body has the following four structures.

The first replication strand corresponding to the elongated strand of the first primer forms the first stem-and-loop structural body which comprises a first double strand region and a first single strand region on one strand, the first double strand region containing a first stem portion, and a first single strand region comprising a first loop portion. The first double strand region contains the first stem portion derived from the S2 sequence and a sequence derived from the complementary sequence of the first template sequence complementary the first stem portion. The first single strand region, which forms the first loop portion, contains an S1 sequence and, optionally, a sequence derived from the complementary sequence of the first template sequence adjacent to a 3' side thereof. Here, in the case where the S2 sequence is designed to be adjacent to a 5' side of the first sequence, the first single strand region, which forms the first loop portion, may not contain a sequence derived from the complementary sequence of the first template sequence on a 3' side of the S1 sequence.

The second replication strand corresponding to the elongated strand of the first primer forms the second stem-and-loop structural body which comprises a second double strand region and a second single strand region on one strand, the second double strand region containing a second stem portion, and the second single strand region containing a second loop portion. The second double strand region contains the second stem portion derived from the complementary sequence of an S4 sequence and a sequence derived from the complementary sequence of the first template sequence complementary thereto. The second single strand region, which forms the second loop portion, contains the complementary sequence of the S3 sequence and, optionally, a sequence derived from the complementary sequence of the first template sequence adjacent to a 5' side thereof. Here, in the case where the S4 sequence is designed to be adjacent to a 5' side of the third sequence, the second single strand region, which forms the second loop portion, may not contain a sequence derived from the complementary sequence of the first template sequence on the 5' side of the S3 sequence.

The third replication strand corresponding to the elongated strand of the second primer forms the third stem-and-loop structural body which comprises a third double strand region and a third single strand region on one strand, the third double strand region containing a third stem portion and the third single strand region containing a third loop portion. The third double strand region contains the third stem portion derived from the S4 sequence and a sequence derived from the complementary sequence of the second template sequence complementary to the third stem portion. The third single strand region, which forms the third loop portion, contains the S3 sequence and, optionally, a sequence derived from the complementary sequence of the second template sequence adjacent to a 3' side thereof. Here, in the case where the S4 sequence is designed to be adjacent to a 5' side of the third sequence, the third single strand region, which forms the third loop portion, may not contain a sequence derived from the complementary sequence of the second template sequence on the 3' side of the S3 sequence.

The fourth replication strand corresponding to the elongated strand of the second primer forms the fourth stem-and-loop structural body which comprises a fourth double strand region and a fourth single strand region on one strand, the fourth double strand region containing a fourth stem portion and a fourth single strand region containing a fourth loop portion. The fourth double strand region contains the fourth stem portion derived from the complementary sequence of the S2 sequence and a sequence derived from the complementary sequence of the second template sequence complementary to the fourth stem portion. The fourth single strand region, which forms the fourth loop portion, contains the complementary sequence of the S1 sequence and, optionally, a sequence derived from the complementary sequence of the second template sequence adjacent to a 5' side thereof. Here, in the case where the S2 sequence is designed to be adjacent to a 5' side of the first sequence, the fourth single strand region, which forms the fourth loop portion, may not contain a sequence derived from the complementary sequence of the second template sequence on the 5' side of the complementary sequence of the S1 sequence.

### <Detection Process>

Subsequent to the amplification of the template nucleic acids, the amplification product obtained in the above-described amplification process is examined as to whether or not an aimed sequence exists in the product or the quantity of the aimed sequence, if exits, is detected.

The detection is carried out using nucleic acid probes. The nucleic acid probes each contains a sequence complementary to a target sequence to be detected. In the nucleic acid analysis method employed here, it suffice if a target sequence to be detected with a nucleic acid probe is designed to be contained in a loop portion of the stem-and-loop structure of the amplification product.

The detection is carried out with hybridization of a nucleic acid probe immobilized on a substrate and the amplification product. After that, the hybridization of the amplification product and the nucleic acid probe is detected with an appropriate detection method.

### <Nucleic Acid Probes>

### [Example 1]

When the primer set described in the items of <Primer Set>, [Example 1] discussed above is used, two types of nucleic acid probes such as of the following, that is, the first nucleic acid probe and/or the second nucleic acid probe may be used.

The first nucleic acid probe may contain a sequence of at least a part of the first single strand region forming the first loop portion of the first replication strand. This first nucleic acid probe, optionally, may further contain, on a 5' side of the first nucleic acid probe, a sequence complementary to a sequence of at least a part of a sequence derived from the complementary sequence of the first template sequence, which is adjacent to a 3' side of the part of the first single strand region. Alternatively, the first nucleic acid probe may contain a sequence of at least a part of the S1 sequence, which is a sequence of at least a part of the first single strand region forming the first loop portion of the first replication strand. This first nucleic acid probe, optionally, may further contain, on a 5' side of the first nucleic acid probe, a sequence complementary to a sequence of at least a part of the sequence derived from the complementary sequence of the first template sequence, which is adjacent to a 3' side of the part of the first single strand region. Here, in regarding to the first primer, in the case where the S2 sequence is designed to be adjacent to the 5' side of the first sequence, a sequence complementary to a sequence of at least a part of the sequence derived from the complementary sequence of the first template sequence may not be contained.

The second nucleic acid probe may contain a sequence of at least a part of the second single strand region forming the second loop portion of the second replication strand. This second nucleic acid probe, optionally, may further contain, on a 3' side of the second nucleic acid probe, a sequence complementary to a sequence of at least a part of a sequence derived from the complementary sequence of the second template sequence, which is adjacent to a 5' side of the part of the second single strand region. Alternatively, the second nucleic acid probe may contain a sequence of at least a part of the complementary sequence of the S1 sequence (that is, the S1' sequence), which is a sequence of at least a part of the second single strand region forming the second loop portion of the second replication strand. This second nucleic acid probe, optionally, may further contain, on a 3' side of the second nucleic acid probe, a sequence complementary to a sequence of at least a part of the sequence derived from the complementary sequence of the second template sequence, which is adjacent to a 5' side of the part of the second single strand region. Here, in regarding to the first primer, in the case where the S2 sequence is designed to be adjacent to the 5' side of the first sequence, a sequence complementary to a sequence of at least a part of the sequence derived from the complementary sequence of the second template sequence may not be contained.

### [Example 2]

When the primer set described in the items of <Primer Set>, [Example 1] discussed above is used, for example, at least one of the group of four types of nucleic acid probes such as of the following, that is, the first nucleic acid probe, the second nucleic acid, the third nucleic acid and the fourth nucleic acid probe may be selected and used.

The first nucleic acid probe may contain a sequence of at least a part of the first single strand region forming the first loop portion of the first replication strand. This first nucleic acid probe, optionally, may further contain, on a 5' side of the first nucleic acid probe, a sequence complementary to a sequence of at least a part of the sequence derived from the complementary sequence of the first template sequence, which is adjacent to a 3' side of the part of the sequence of the first single strand region. Alternatively, the first nucleic acid probe may contain a sequence of at least a part of the complementary sequence of the S1 sequence, which is a sequence of at least a part of the first single strand region comprising the first loop portion of the first replication strand. This first nucleic acid probe, optionally, may further contain, on a 5' side of the first nucleic acid probe, a sequence complementary to a sequence of at least a part of a sequence derived from the complementary sequence of the first template sequence, which is adjacent to a 3' side of the part of the first single strand region. Here, in the case where the S2 sequence is designed to be adjacent to the 5' side of the first sequence, a sequence complementary to a sequence of at least a part of the sequence derived from the complementary sequence of the first template sequence may not be contained.

The second nucleic acid probe may contain a sequence of at least a part of the second single strand region forming the second loop portion of the second replication strand. This second nucleic acid probe, optionally, may further contain, on a 3' side of the second nucleic acid probe, a sequence complementary to a sequence of at least a part of a sequence derived from the complementary sequence of the first template sequence, which is adjacent to a 5' side of the part of the second single strand region. Alternatively, the second nucleic acid probe may contain a sequence of at least a part of the sequence of the S3 sequence, which is a sequence of at least a part of the second single strand region forming the second loop portion of the second replication strand. This second nucleic acid probe, optionally, may further contain, on a 3' side of the second nucleic acid probe, a sequence complementary to a sequence of at least a part of a sequence derived from the complementary sequence of the first template sequence, which is adjacent to a 5' side of the part of the second single strand region. Here, in the case where the S4 sequence is designed to be adjacent to the 5' side of the third sequence, a sequence complementary to a sequence of at least a part of the sequence derived from the complementary sequence of the first template sequence may not be contained.

The third nucleic acid probe may contain a sequence of at least a part of the third single strand region forming the third loop portion of the third replication strand. This third nucleic acid probe, optionally, may further contain, on a 5' side of the third nucleic acid probe, a sequence complementary to a sequence of at least a part of a sequence derived from the complementary sequence of the second template sequence, which is adjacent to a 3' side of the part of the sequence of the third single strand region. Alternatively, the third nucleic acid probe may contain a sequence of at least a part of the complementary sequence of the S3 sequence, which is a sequence of at least a part of the third single strand region forming the third loop portion of the third replication strand. This third nucleic acid probe, optionally, may further contain, on a 5' side of the third nucleic acid probe, a sequence complementary to a sequence of at least a part of a sequence derived from the complementary sequence of the second template sequence, which is adjacent thereto on a 3' side of the part of the sequence. Here, in the case where the S4 sequence is designed to be adjacent to the 5' side of the third sequence, a sequence complementary to a sequence of at least a part of the sequence derived from the complementary sequence of the second template sequence may not be contained.

The fourth nucleic acid probe may contain a sequence of at least a part of the fourth single strand region forming the fourth loop portion of the fourth replication strand. This fourth nucleic acid probe, optionally, may further contain, on a 3' side of the fourth nucleic acid probe, a sequence complementary to a sequence of at least a part of a sequence derived from the complementary sequence of the second template sequence, which is adjacent to a 5' side of the part of the fourth single strand region. Alternatively, the fourth nucleic acid probe may contain a sequence of at least a part of the S1 sequence, which is a sequence of at least a part of the fourth single strand region forming the fourth loop portion of the fourth replication strand. This fourth nucleic acid probe, optionally, may further contain, on a 3' side of the fourth nucleic acid probe, a sequence complementary to a sequence of at least a part of a sequence derived from the complementary sequence of the second template sequence, which is adjacent to a 5' side of the part of the fourth single strand region. Here, in the case where the S2 sequence is designed to be adjacent to the 5' side of the first sequence, a sequence complementary to a sequence of at least a part of the sequence derived from the complementary sequence of the second template sequence may not be contained.

### <DNA Chip>

The DNA chip used in this embodiment may comprise a substrate and a nucleic acid probe immobilized on the substrate. The substrate of the DNA chip may be of any conventionally known type employable for microarrays, such as an electrochemical detection type, a typical example of which is a current detection type, fluorescent detection type, chemical coloring type or radioactivity detection type.

Any type of microarray can be manufactured by any method conventionally known by itself. For example, in the case of a current detection type microarray, the negative control probe immobilization region and the detection prove immobilization region may be located on respective electrodes different from each other.

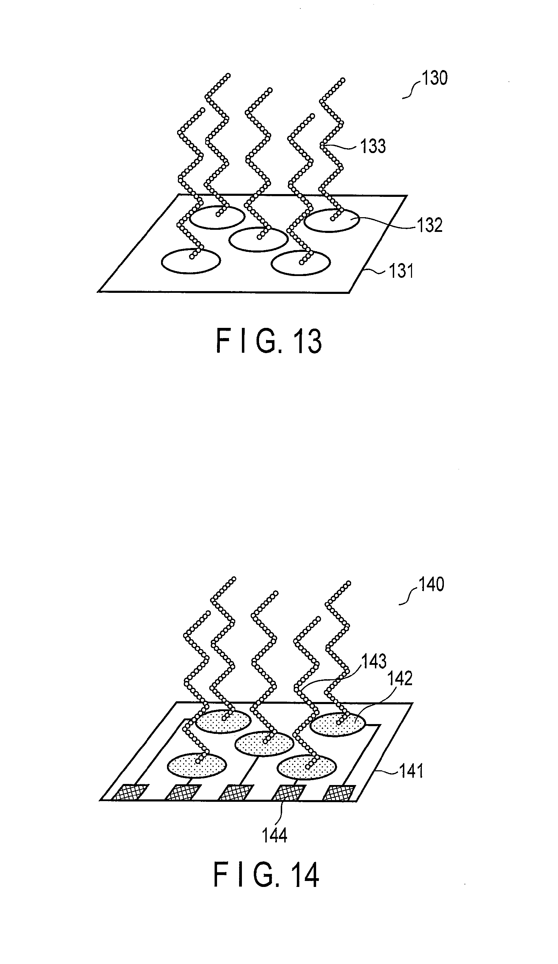

An example of the DNA chip is shown schematically in FIG. 13, but the embodiments are not limited to this. A DNA chip 130 comprises a substrate 131 and immobilization regions 132 formed thereon. A nucleic acid probe 133 is immobilized onto each of the immobilization regions 132. This DNA chip 130 can be manufactured using a method conventionally known in the field of the embodiments. The number of immobilization regions 132 located on the substrate 131 and the arrangement thereof can be changed appropriately by a person having ordinary skill in the art as required. Such a DNA chip may be used suitably for a detection method which uses fluorescence.

Another example of the DNA chip is shown in FIG. 14. A DNA chip 140 shown in FIG. 14 comprises a substrate 141 and electrodes 142 formed thereon. A nucleic acid probe 143 is immobilized onto each of the electrodes 142. Each of the electrodes 142 is connected to each respective pad 144. Electrical data from each electrode 142 is obtained via each respective pad 144. This DNA chip 140 can be manufactured using a method conventionally known in the field of the embodiments. The number of immobilization regions 142 located on the substrate 141 and the arrangement thereof can be changed appropriately by a person having ordinary skill in the art as required. Further, the DNA chip of this embodiment may comprise a reference electrode and a counter electrode as required.

For the electrodes, gold, an alloy of gold, a metal element of silver, platinum, nickel, palladium, silicon, germanium, gallium and tungsten and any alloy of these, carbons or oxides or compounds thereof, such as graphite and glassy carbon, can be used thought the embodiments are not limited to these.

Such a DNA chip as of this embodiment may be used suitably for an electrochemical detection method.

### <Hybridization Conditions>

The hybridization may be carried out under conditions appropriate for fully forming hybrids. The appropriate conditions may differ from one case to another depending on the type and structure of the target nucleic acid, the type of bases contained in the target sequence and the type of nucleic acid probes. For example, the hybridization may be carried out in a buffer liquid having an ionic strength within a range of 0.01 to 5 and a pH in a rage of 5 to 9. The reaction temperature may be in a range of 10°C to 90°C. Stirring, shaking or the like may be carried out to increase the reaction efficiency. To the reaction solution, dextran sulfate, a hybridization promoter such as DNA of sermon sperms or DNA of bovine thymus gland, EDTA, a surfactant or the like may be added.

### <Washing Conditions>

For the washing liquid for washing the DNA chip after the hybridization, a buffer liquid having an ionic strength within a range of 0.01 to 5 and a pH in a rage of 5 to 9 may be appropriately used. The washing liquid should preferably contain salt, a surfactant or the like. For example, an SSC solution prepared using sodium chloride or sodium citrate, a Tris-HCl solution, a Tween20 solution, an SDS solution or the like can be appropriately used. The washing is carried out at a temperature in a range of, for example, 10°C to 90°C. The washing liquid is allowed to pass or reside on the surface of the probe-immobilized substrate or the regions where nucleic probes are immobilized. Alternatively, the DNA chip may be immersed in the washing liquid. In this case, the washing liquid should preferably be contained in a temperature-controllable container.

### <Detection Method>

For the detection of a hybrid generated by the hybridization process, the fluorescent detection type or electrochemical detection type can be utilized.

### (a) Fluorescent detection type

For the detection, a fluorescent labeling substance is used. The primer used in the nucleic acid amplification process may be labeled with a fluorescently active substance such as a fluorescent dye, namely, FITC, Cy3, Cy5 or rhodamine. Alternatively, a second probe labeled with any of these substances may be used. A plurality of labeling substances may be used at the same time. With the detection device, a labeled sequence or a label in the secondary probe is detected. An appropriate detection device should be used according to the label employed. For example, a fluorescent substance is used as the label, the fluorescent detector should be used for the detection.

### (a) Electrochemical detection type

A double strand recognition substance conventionally known in this field is used. The double strand recognition substance may be selected from Hoechst 33258, acridine orange, quinacrine, daunomycin, metallointercalator, bis-intercalator such as bis acridine, a tris-intercalator and a poly-intercalator. Further, these double strand recognition substance may be modified with an electrochemically active metal complex such as ferrocene or viologen.

The double strand recognition substances, although depending on the type, are generally used at a concentration in a range of 1ng/mL to 1mg/mL. In this occasion, a buffer liquid having an ionic strength within a range of 0.001 to 5 and a pH in a rage of 5 to 10 can be used.

For the measurement, for example, a potential equal to or higher than the potential with which the double strand recognition substance electrochemically reacts is applied, and thus the reaction current value derived from the double strand recognition substance is measured. In this occasion, the potential may be applied at a constant rate or in pulses, or a constant potential may be applied. Such devices as a potentiostat, digital multimeter and function generator may be used to control the current and voltage. The electrochemical detection can be carried out with any conventionally known method in this field.

### <Assay Kit>

The embodiments may be in the form of an assay kit. The assay kit may contain a primer set and a nucleic acid primer of any of the above-described embodiments, and optionally it may comprise a dilution liquid, various types of enzymes, a reaction container, an instruction manual and the like.

### Example

### Example 1

An example which illustrates a method of detecting a product amplified with use a loop-forming primer by using a DNA chip, will now be described in detail.

### (1) Amplification

### <Template>

As the template, a plasmid to which a Bacillus subtilis sequence is introduced was used. The sequence of Bacillus subtilis is shown in SEQ ID NO. 1.

### <Primers>

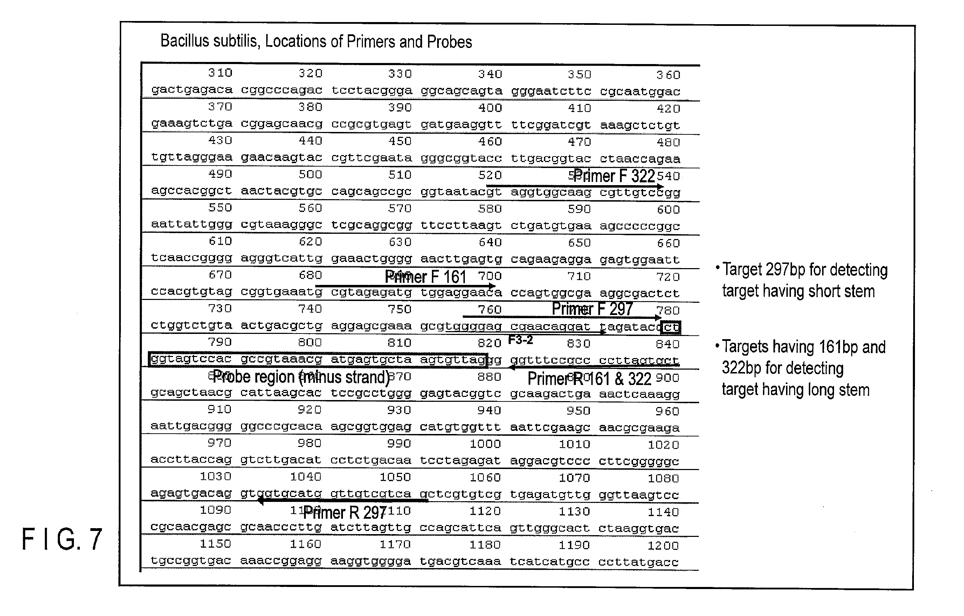

As normal PCR primers for amplifying 297bp, 5'-GGGAGCGAACAGGATTAGATACC-3' (F primer: SEQ ID NO. 2) and 5'-CTGACGACAACCATGCACC-3' (R primer: SEQ ID NO. 3), and as normal PCR primers for amplifying 322bp, 5'-GTAGGTGGCAAGCGTTGTCC-3 (F primer: SEQ ID NO. 4) and 5'-AGCACTAAGGGGCGGAAACC-3' (R primer: SEQ ID NO. 5) were prepared. The positions for the primers are shown in FIG. 7. Further, as loop-forming primers for the amplification of a 297bp target which form a stem-and-loop-structure with a short stem, 5'-CCCTAACACTTAGCACTCATCGTT-GGGAGCGAACAGGATTAGATACC-3' (F primer: SEQ ID NO. 6), 5'-GAAACCCCCTAACACTTAGC-GGGAGCGAACAGGATTAGATACC-3' (F primer: SEQ ID NO. 7) and 5'-AGCACTAAGGGGCGGAAACC-GGGAGCGAACAGGATTAGATACC-3' (F primer: SEQ ID NO. 8) were prepared.

As an R primer, that similar to a normal PCR primer (SEQ ID NO. 3) was used. Further, as loop-forming primers for the amplification of a 322bp target which form a stem-and-loop-structure with a long stem, 5'-GGTAGTCCACGCCGTAAACG-AGCACTAAGGGGCGGAAACC-3' (R primer: SEQ ID NO. 9), 5'-ATTAGATACCCTGGTAGTCCAC-AGCACTAAGGGGCGGAAACC-3' (R primer: SEQ ID NO. 10), 5'-GGAGCGAACAGGATTAGATACCCT-AGCACTAAGGGGCGGAAACC-3 (R primer: SEQ ID NO. 11) were prepared, and as an F primer, that similar to a normal PCR primer (SEQ ID NO. 4) was used. The lengths of single strand loops obtained in regarding to SEQ ID NOS. 6, 7, 8, 9 10 and 11 are 42bp, 52bp, 66bp, 40bp, 50bp and 60bp, respectively.

### <Amplification>

The amplification was carried out with KAPA2G Fast DNA Kit (a product of KAPA Biosystems), in which a thermal denaturation was carried out at 95°C for 1 minute and after that, a cycle having conditions of 95°C for 5 seconds and 65°C for 5 seconds were repeated 40 cycles. The amplification device was GeneAmp PCR9700 (a product of Applied Biosystems). The amplification time was about 47 minutes. After the amplification, the amplification product was confirmed by electrophoresis.

### (2) Chip Detection

### <Detection Probes>

For the chip detection of the amplification product of Bacillus subtilis, obtained in (1), two types of minus strand probes having different lengths, namely 5'-ACTTAGCACTCATCGTTTACGGCGTGGACT-3' (SEQ ID NO. 17) and 5'-CTAACACTTAGCACTCATCGTTTACGGCGTGGACTACCAG-3' (SEQ ID NO. 18) were prepared. The positions for the probes are shown in FIG. 7. In addition, as a negative control, 5'-TGCTTCTACACAGTCTCCTGTACCTGGGCA-3' (SEQ ID NO. 19), which has a sequence irrelevant to that of Bacillus subtilis was prepared. The above-mentioned probes were each subjected to modification of 3' terminal end side thereof with thiol in order for immobilization to gold electrode.

### <Manufacture of Probe-Immobilized Electrode>

The immobilization of each probe was carried out by utilizing the strong covalency between thiol and gold. That is, with a probe solution containing a 1mM-mercaptohexanol solution, a gold electrode is spotted, and then the spot was dried. For each of the identical probes, four electrodes were subjected to the spotting. After dried, the subject was washed with pure water, dried with wind, and thus probe-immobilized electrodes were obtained.

### <Hybridization>

The amplification product obtained in (1) was subjected to thermal denaturation at 95°C for 5 minutes and after that, a salt of 2×SSC was added. The thus obtained solution is used for the immersion of the probe-immobilized electrodes manufactured as above, and let stand still at 64°C for 5 minutes for hybridization. After that, the probe-mobilized electrodes were immersed in a 0.2×SSC solution at 25°C for 1 minute, followed by washing. Subsequently, the probe-mobilized electrodes were immersed in a PIPES buffer solution containing 50µM of Hoechst 33258, which is an intercalating agent, for 1 minute. After that, the oxidation current response of the Hoechst 33258 solution was measured.

### (3) Results

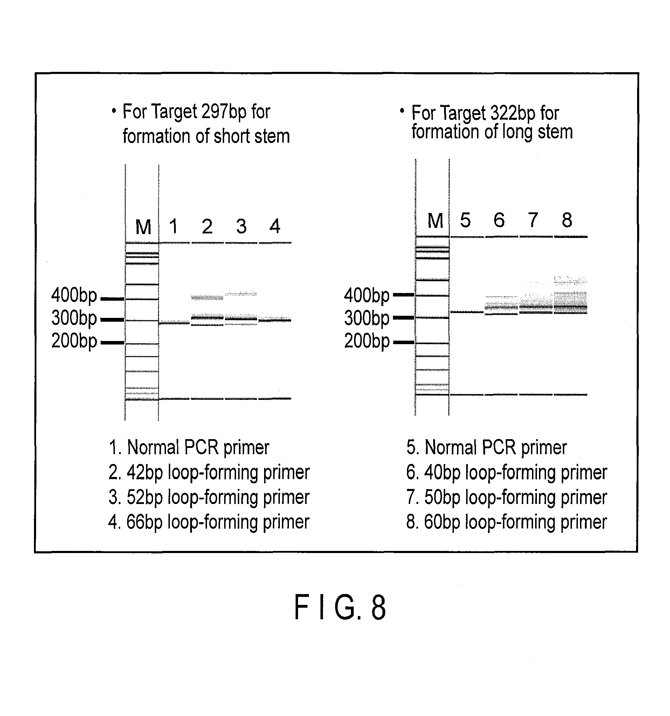

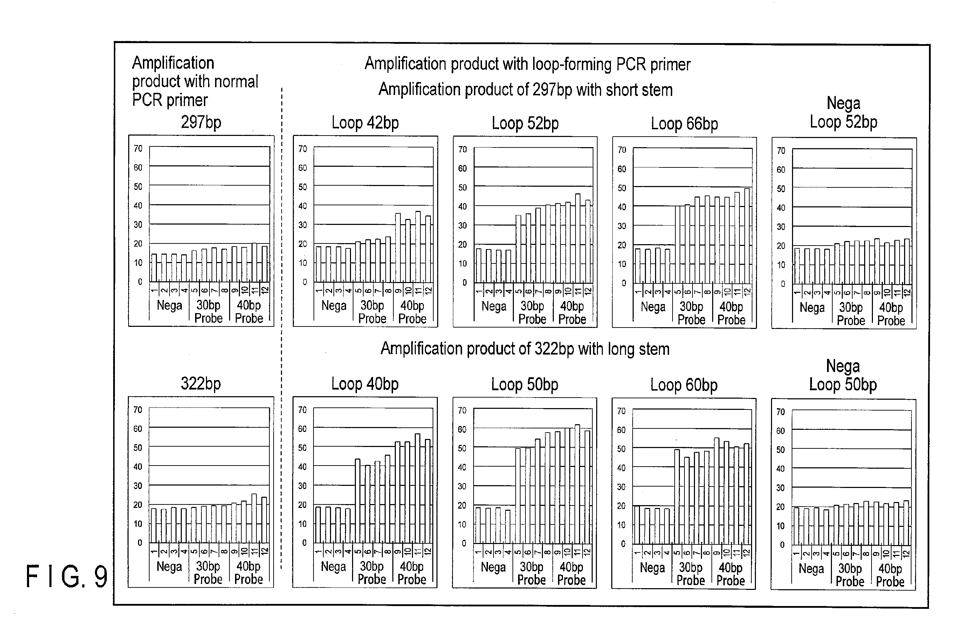

The results of the electrophoresis are shown in FIG. 8. As to the products amplified with the normal PCR primers and those amplified with the loop-forming primers, in any cases, the bands were confirmed in the target regions. In connection with the products of the loop-forming primers, there were a plurality of bands, which suggests that products of a plurality of target structures had been formed. FIG. 9 shows the results of the chip detection. As to the products amplified with the normal PCR primers, a signal increase was not observed. On the other hand, as to the products amplified with the loop-forming primers, a signal increase was observed. Further, a high signal increase was detected more stably in the case where amplification products having a long stem portion are detected, as compared to the case of the amplification products having a short stem portion. In terms of the loop length, a sufficient signal increase was confirmed from 40bp to 60bp. Further, as to the products of the negative control, to which TE was added in place of the template, it was confirmed that there were no signal increases.

### Example 2

Discussion on further modified examples which involve different target lengths and different high-rate PCR enzymes, will now be provided in connection the method of detecting a product amplified with use loop-forming primers by using a DNA chip. Further, the case where both of the F primers and R primers were used as the loop-forming primers was also discussed.

### (1) Amplification

### <Template>

A template similar to that used in Example 1 was used here. <Primers>

In order to obtain targets which give different target lengths each other and which show stem-and-loop structures having long stems, 5'-GCGTAGAGATGTGGAGGAAC-3' (F primer: SEQ ID NO. 12) was prepared for targeting 161bp, and further 5'-GTAGGTGGCAAGCGTTGTCC-3' (F primer: SEQ ID NO. 4) for targeting 322bp, 5'-AGGCAGCAGTAGGGAATCTTCC-3' (F primer: SEQ ID NO. 13) for targeting 511bp and 5'-CCTGTAAGACTGGGATAACTCCG-3' (F primer: SEQ ID NO. 14) for targeting 734bp were prepared. Further, in order to obtain products for the case where both of the F primers and R primers were used as the loop-forming primers, 5'-GCTCCTCAGCGTCAGTTACA-GCGTAGAGATGTGGAGGAAC-3' (F primer: SEQ ID NO. 15) was prepared for targeting 161bp, and further 5'-GCTTTCACATCAGACTTAAGGAACC-GTAGGTGGCAAGCGTTGTCC-3' (F primer: SEQ ID NO. 4) for targeting 322bp. The R primer used was common to all cases, which was 5'-ATTAGATACCCTGGTAGTCCAC-AGCACTAAGGGGCGGAAACC-3' (SEQ ID NO. 10).

### <Amplification>

The amplification was carried out with KAPA2G Fast used in (1), Takara SpeedSTAR™ HS DNA Polymerase (a product of TAKARA Bio), and Phire Hot Start II DNA Polymerase (a product of Thermo Fisher Scientific).

The reaction conditions employed here were similar to those of the method used in Example 1.

### (2) Chip Detection

The chip detection was carried out in a similar manner to that of Example 1 except that the thermal treatment of the amplification products carried out before the hybridization was omitted.

### (3) Results

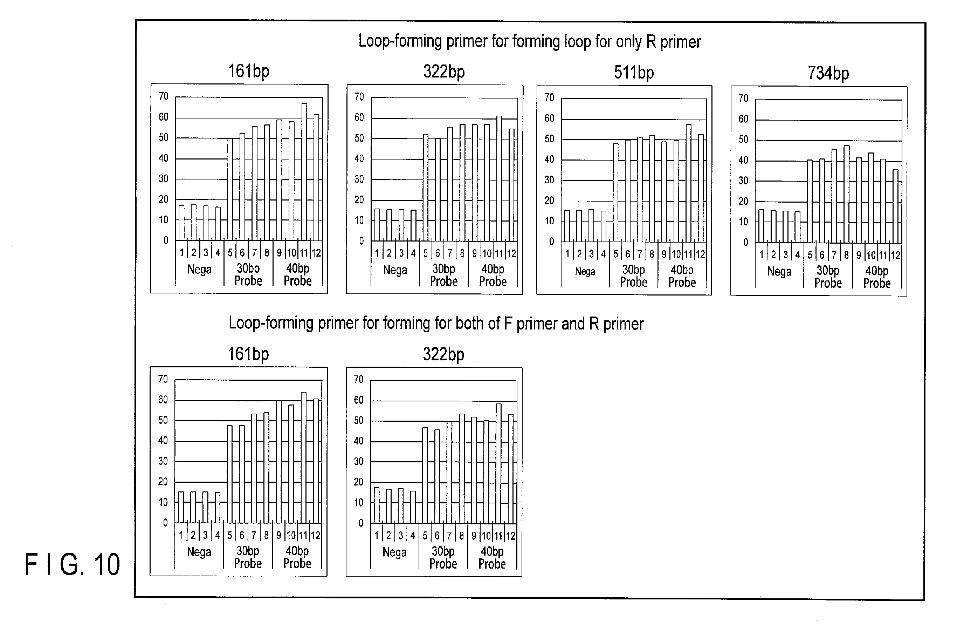

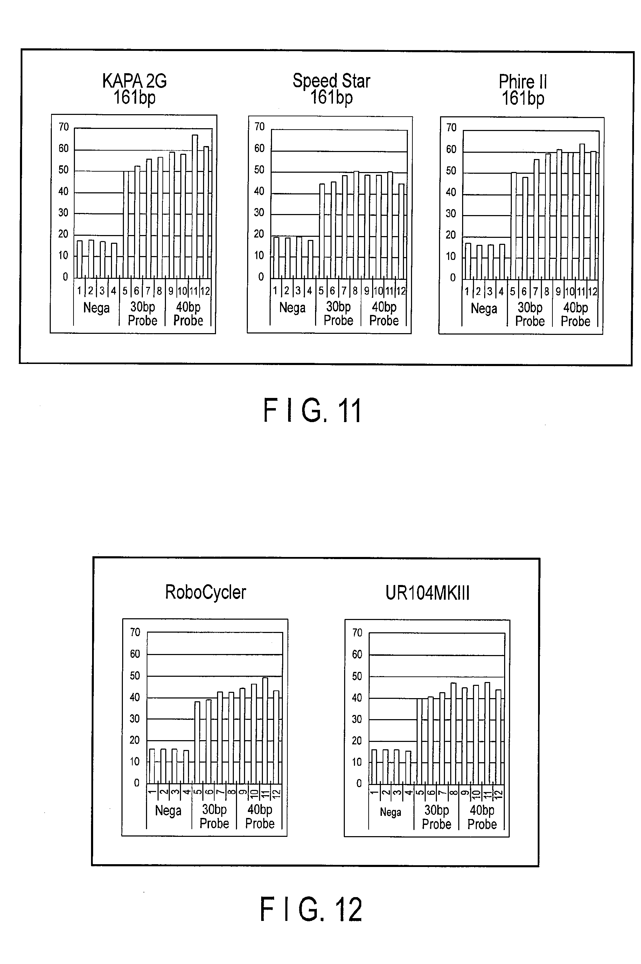

The results of the detection of the amplification products with different target lengths each other are shown in FIG. 10. Such a tendency was observed that as the target length is larger, the signal increase is slightly lowered, but in each case, a sufficient signal increase was confirmed. When the both of the F primers and R primers were used as loop-forming primers, in each case, a sufficient signal increase was confirmed. Further, as shown in FIG. 11. a signal increase was confirmed in all of the 3 types of high-rate PCR enzymes examined.

### Example 3

Discussion on further modified examples will now be provided in connection the method of detecting a product amplified with use a loop-forming primer by using a DNA chip, in which rotary PCR apparatuses of RoboCycler Gradient 96 (a product of Stratagene) and UR104MKIII (a product of Trust Medical) were used to shorten the time required for amplification.

### (1) Amplification

### <Template>

A template similar to that used in Example 1 was used here. <Primers>

To obtain a taget of 166bp having a stem-and-loop structure having a long stem by amprification, as a loop-forming primer, 5'-GCGTAGAGATGTGGAGGAAC-3' (F primer: SEQ ID NO. 12) and 5'-ATTAGATACCCTGGTAGTCCAC-AGCACTAAGGGGCGGAAACC-3' (R primer: SEQ ID NO. 10) were used.

### <Amplification>

The amplification was carried out with a KAPA2G Fast DNA Kit. In the case of RoboCycler Gradient 96, a thermal denaturation was carried out at 97°C for 1 minute and after that, a cycle having conditions of 97°C for 10 seconds and 53°C for 10 seconds were repeated 40 cycles. Here, the amplification time was about 18 minutes and 30 seconds. In the case of UR104MKIII, the amount of enzyme was set to be twice as large as that of the recommended concentration in the composition. Here, a thermal denaturation was carried out at 95°C for 1 minute and after that, 95°C for 4 seconds to 65°C for 7 seconds were carried out over 40 cycles. The amplification time was about 10 minutes and 30 seconds.

### (2) Chip Detection

The chip detection was carried out in a similar manner to that of Example 2.

### (3) Results

As shown in FIG. 12, it was found that the products amplified with RoboCycler Gradient 96 and UR104MKIII can be subjected to chip detection.

The sequence of Bacillus subtilis, used as the template sequence in Examples 1 to 3 discussed above is shown in TABLE 1.
[TABLE 1]

**Table 1**

| |
|---|
| SEQ ID No. 1 Sequence of bacillus subtilis |
| AGGACGAACGCTGGCGGCGTGCCTAATACATGCAAGTCGAGCGGACAGATGGGAG CTTGCTCCCTGATGTTAGCGGCGGACGGGTGAGTAACACGTGGGTAACCTGCCTG TAAGACTGGGATAACTCCGGGAAACCGGGGCTAATACCGGATGCTTGTTTGAACC GCATGGTTCAAACATAAAAGGTGGCTTCGGCTACCACTTACAGATGGACCCGCGG CGCATTAGCTAGTTGGTGAGGTAATGGCTCACCAAGGCAACGATGCGTAGCCGAC CTGAGAGGGTGATCGGCCACACTGGGACTGAGACACGGCCCAGACTCCTACGGGA GGCAGCAGTAGGGAATCTTCCGCAATGGACGAAAGTCTGACGGAGCAACGCCGCG TGAGTGATGAAGGTTTTCGGATCGTAAAGCTCTGTTGTTAGGGAAGAACAAGTAC CGTTCGAATAGGGCGGTACCTTGACGGTACCTAACCAGAAAGCCACGGCTAACTA CGTGCCAGCAGCCGCGGTAATACGTAGGTGGCAAGCGTTGTCCGGAATTATTGGG CGTAAAGGGCTCGCAGGCGGTTCCTTAAGTCTGATGTGAAAGCCCCCGGCTCAAC CGGGGAGGGTCATTGGAAACTGGGAACTTGAGTGCAGAAGAGGAGAGTGGAATT CCACGTGTAGCGGTGAAATGCGTAGAGATGTGGAGGAACACCAGTGGCGAAGGCG ACTCTCTGGTCTGTAACTGACGCTGAGGAGCGAAAGCGTGGGGAGCGAACAGGAT TAGATACCCTGGTAGTCCACGCCGTAAACGATGAGTGCTAAGTGTTAGGGGGTTT CCGCCCCTTAGTGCTGCAGCTAACGCATTAAGCACTCCGCCTGGGGAGTACGGTC GCAAGACTGAAACTCAAAGGAATTGACGGGGGCCCGCACAAGCGGTGGAGCATGT GGTTTAATTCGAAGCAACGCGAAGAACCTTACCAGGTCTTGACATCCTCTGACAA TCCTAGAGATAGGACGTCCCCTTCGGGGGCAGAGTGACAGGTGGTGCATGGTTGT CGTCAGCTCGTGTCGTGAGATGTTGGGTTAAGTCCCGCAACGAGCGCAACCCTTG ATCTTAGTTGCCAGCATTCAGTTGGGCACTCTAAGGTGACTGCCGGTGACAAACC GGAGGAAGGTGGGGATGACGTCAAATCATCATGCCCCTTATGACCTGGGCTACAC ACGTGCTACAATGGACAGAACAAAGGGCAGCGAAACCGCGAGGTTAAGCCAATCC CACAAATCTGTTCTCAGTTCGGATCGCAGTCTGCAACTCGACTGCGTGAAGCTGG AATCGCTAGTAATCGCGGATCAGCATGCCGCGGTGAATACGTTCCCGGGCCTTGT ACACACCGCCCGTCACACCACGAGAGTTTGTAACACCCGAAGTCGGTGAGGTAAC CTTTTAGGAGCCAGCCGCCGAAGGTGGGACAGATGATTGGGGTGAAGTCGTAACA AGGTAGCCGTATCGGAAGGTGC |

The sequence of each of the primers used in Examples 1 to 3 discussed above is shown in TABLE 2.
[TABLE 2]

**Table 2**

| SEQ ID No | Name | Sequence |
|---|---|---|
| 2 | Normal PCR F primer 297 bp | GGGAGCGAACAGGATTAGATACC |
| 3 | Normal PCR R primer 297 bp | *CTGACGACAACCATGCACC* |
| 4 | Normal PCR F primer 322 bp | GTAGGTGGCAAGCGTTGTCC |
| 5 | Normal PCR R primer 322 bp | *AGCACTAAGGGGCGGAAACC* |
| 6 | F primer for loop-forming target 42mer having a short stem | *CCCTAACACTTAGCACTCATCGTT*-GGGAGCGAACAGGATTAGATACC |
| 7 | F primer for loop-forming target 52mer having a short stem | *GAAACCCCCTAACACTTAGC-*GGGAGCGAACAGGATTAGATACC |
| 8 | F primer for loop-forming target 66mer having a short stem | *AGCACTAAGGGGCGGAAACC*-GGGAGCGAACAGGATTAGATACC |
| 9 | R primer for loop-forming target 40mer having a long stem | GGTAGTCCACGCCGTAAACG-*AGCACTAAGGGGCGGAAACC* |
| 10 | R primer for loop-forming target 50mer having a long stem | ATTAGATACCCTGGTAGTCCAC-*AGCACTAAGGGGCGGAAACC* |
| 11 | R primer for loop-forming target 60mer having a long stem | GGAGCGAACAGGATTAGATACCCT-*AGCACTAAGGGGCCGGAAACC* |
| 12 | Normal PCR F primer 161bp | GCGTAGAGATGTGGAGGAAC |
| 13 | Normal PCR F primer 511bp | AGGCAGCAGTAGGGAATCTTCC |
| 14 | Normal PCR F primer 734bp | CCTGTAAGACTGGGATAACTCCG |
| 15 | F primer for both loops-forming target 161bp | *GCTCCTCAGCGTCAGTTACA*-GCGTAGAGATGTGGAGGAAC |
| 16 | F primer for both loops-forming target 322bp | *GCTTTCACATCAGACTTAAGGAACC*-GTAGGTGGCAAGCGTTGTCC |

The sequence of the probes used in Examples 1 to 3 discussed above is shown in TABLE 3.
[TABLE 3]
[Sequence Table]

**Table 3**

| SEQ ID No | Name | Sequence |
|---|---|---|
| 17 | R probe 30mer for detecting bacillus subtilis | ACTTAGCACTCATCGTTTACGGCGTGGACT |
| 18 | R probe 40mer for detecting bacillus subtilis | CTAACACTTAGCACTCATCGTTTACGGCGTGGACTACCAG |
| 19 | Negative control probe | TGCTTCTACACAGTCTCCTGTACCTGGGCA |

## Claims

1. A method of analyzing a nucleic acid in a sample,
the nucleic acid to be amplified comprising a first template nucleic acid and a second template nucleic acid,
the first template nucleic acid comprising a first sequence located on a 3' terminal, a first template sequence adjacent to a 5' side of the first sequence, and a second sequence adjacent to a 5' side of the first template sequence and located on the 5' terminal, and
the second template nucleic acid, which is a strand complementary to the first template nucleic acid, comprising a third sequence located on a 3' terminal thereof, a second template sequence adjacent to a 5' side of the third sequence, and a fourth sequence adjacent to a 5' side of the second template sequence and located on the 5' terminal,
the method comprising:
(a-1) preparing a first primer and a second primer configured to amplify the first template nucleic acid and the second template nucleic acid,
wherein the first primer comprises an S1 sequence complementary to the first sequence and an S2 sequence joined to a 5' terminal side of the S1 sequence, the S2 sequence is a sequence, which is same as a sequence of at least a part of the first template sequence, located adjacent to or in a vicinity of a 5' side of the first sequence on the first template nucleic acid and
the second primer comprises an S3 sequence complementary to the third sequence; and
a first replication strand corresponding to an elongated strand from the first primer forms a first stem-and-loop structural body, the first stem-and-loop structural body comprises a first double strand region and a first single strand region on one strand, the first double strand region comprises a first stem portion derived from the S2 sequence and a sequence derived from a complementary sequence of the first template sequence, the sequence derived from a complementary sequence of the first template sequence and the first stem portion in the first double strand region are complementary each other, the first single strand region forms a first loop portion, and the first single strand region, which forms the first loop portion, comprises the S1 sequence; and
a second replication strand corresponding to an elongated strand from the second primer forms a second stem-and-loop structural body, the second stem-and-loop structural body comprises a second double strand region and a second single strand region on one strand, the second double strand region comprises a second stem portion derived from a complementary sequence of the S2 sequence and a sequence complementary to the second template sequence, the sequence complementary to the second template sequence and the second stem portion in the second double strand region are complementary each other, the second single strand region forms a second loop portion, and the second single strand region, which forms the second loop portion, comprises the S1 sequence;
(a-2) preparing a first nucleic acid probe and/or a second nucleic acid probe configured to detect the first replication strand and/or the second replication strand,
wherein the first nucleic acid probe comprises a sequence of at least a part of the first single strand region forming the first loop portion of the first replication strand, and the second nucleic acid probe comprises a sequence of at least a part of the second single strand region forming the second loop portion of the second replication strand;
(b) amplifying the sample using the first primer and the second primer,
(c) reacting an amplification product obtained in (b) with the first nucleic acid probe and/or the second nucleic acid probe,
(d) detecting presence/absence of a hybridization and/or measuring a quantity of hybridization between the first nucleic acid probe and/or the second nucleic acid probe and the amplification product, and
(e) determining presence or absence of the target nucleic acid in the sample and/or determining a quantity of target nucleic acid presence, based on a result in (d).

2. The nucleic acid analysis method of claim 1, wherein the amplification is by a PCR amplification reaction.

3. The nucleic acid analysis method of claim 2, wherein the nucleic acid probe is immobilized on a substrate.

4. The nucleic acid analysis method of claim 3, wherein the detecting the hybridization and/or measuring a quantity of the hybridization between the nucleic acid probe immobilized on the substrate and the amplification product is carried out using an intercalating agent which indicates an electrochemical activity.

5. The nucleic acid analysis method of claim 4, wherein the sample comprises a gene and an object of the analysis is the gene.

6. An assay kit used for the nucleic acid analysis method of claim 1, the assay kit comprising:
a primer set and a nucleic acid probe,
wherein a first template nucleic acid to be amplified with the primer set comprises a first sequence located on a 3' terminal, a first template sequence adjacent to a 5' side of the first sequence, and a second sequence adjacent to a 5' side of the first template sequence and located on the 5' terminal, and
a second template nucleic acid to be amplified with the primer set is a strand complementary to the first template nucleic acid, and comprises a third sequence located on a 3' terminal thereof, a second template sequence adjacent to a 5' side of the third sequence, and a fourth sequence adjacent to a 5' side of the second template sequence and located on the 5' terminal thereof,
the primer set satisfies the following (1) and/or (2):
(1) the first primer comprises an S1 sequence and an S2 sequence, the S1 sequence being complementary to the first sequence, the S2 sequence joined to a 5' terminal side of the S1 sequence, and the S2 sequence being a sequence, which is same as a sequence of at least a part of the first template sequence, located adjacent to or in a vicinity of a 5' side of the first sequence on the first template nucleic acid, and
the second primer comprises an S3 sequence complementary to the third sequence;
(2) the first primer comprises an S1 sequence and an S2 sequence, the S1 sequence being complementary to the first sequence, the S2 sequence joined to a 5' side of the S1 sequence, and the S2 sequence being a sequence, which is same as a sequence of at least a part of the first template sequence, located adjacent to or in a vicinity of a 5' side of the first sequence on the first template nucleic acid, and
the second primer comprises an S3 sequence and an S4 sequence, the S3 sequence being complementary to the third sequence, the S4 sequence joined to a 5' side of the S3 sequence, and the S4 sequence being a sequence, which is same as a sequence of at least a part of the second template sequence, located adjacent to or in a vicinity of a 5' side of the third sequence on the second template nucleic acid;
the nucleic acid probe is at least one type selected from the group consisting of:
a nucleic acid probe comprising a sequence of at least a part of the S1 sequence;
a nucleic acid probe comprising a sequence of at least a part of the complementary sequence of the S1 sequence;
a nucleic acid probe comprising a sequence of at least a part of the S3 sequence; and
a nucleic acid probe comprising a sequence of at least a part of the complementary sequence of the S3 sequence.

7. A method of analyzing nucleic acids in a sample,
the nucleic acids to be amplified comprising a first template nucleic acid and a second template nucleic acid,
the first template nucleic acid comprising a first sequence located on a 3' terminal, a first template sequence adjacent to a 5' side of the first sequence, and a second sequence adjacent to a 5' side of the first template sequence and located on the 5' terminal, and
the second template nucleic acid, which is a strand complementary to the first template nucleic acid, comprising a third sequence located on a 3' terminal thereof, a second template sequence adjacent to a 5' side of the third sequence, and a fourth sequence adjacent to a 5' side of the second template sequence and located on the 5' terminal,
the method comprising:
(a-1) preparing a first primer and a second primer configured to amplify the first template nucleic acid and the second template nucleic acid,
wherein the first primer comprises an S1 sequence complementary to the first sequence and an S2 sequence joined to a 5' terminal side of the S1 sequence, the S2 sequence is a sequence, which is same as a sequence of at least a part of the first template sequence, located adjacent to or in a vicinity of a 5' side of the first sequence on the first template nucleic acid and
the second primer comprises an S3 sequence complementary to the third sequence and an S4 sequence joined to a 5' side of the S3 sequence, the S4 sequence is a sequence, which is same as a sequence of at least a part of the second template sequence, located adjacent to or in a vicinity of a 5' side of the third sequence on the second template nucleic acid;
further, a first replication strand corresponding to an elongated strand from the first primer forms a first stem-and-loop structural body, the first stem-and-loop structural body comprises a first double strand region and a first single strand region on one strand, the first double strand region comprises a first stem portion derived from the S2 sequence and a sequence derived from a complementary sequence of the first template sequence, the sequence derived from a complementary sequence of the first template sequence is complementary to the first stem portion, the first single strand region forms a first loop portion, and the first single strand region, which forms the first loop portion, comprises the S1 sequence;
a second replication strand corresponding to an elongated strand from the first primer forms a second stem-and-loop structural body, the second stem-and-loop structural body comprises a second double strand region and a second single strand region on one strand, the second double strand region comprises a second stem portion derived from a complementary sequence of the S4 sequence and a sequence derived from a complementary sequence of the first template sequence, the sequence derived from a complementary sequence of the first template sequence is complementary to the second stem portion, the second single strand region forms a second loop portion, and the second single strand region, which forms the second loop portion, comprises a complementary sequence to the S3 sequence;
a third replication strand corresponding to an elongated strand from the second primer forms a third stem-and-loop structural body, the third stem-and-loop structural body comprises a third double strand region and a third single strand region on one strand, the third double strand region comprises a third stem portion derived from the S4 sequence and a sequence derived from a complementary sequence of the second template sequence, the sequence derived from a complementary sequence of the second template sequence is complementary to the third stem portion, the third single strand region forms a third loop portion, and the third single strand region, which forms the third loop portion, comprises the S3 sequence; and
a fourth replication strand corresponding to an elongated strand from the second primer forms a fourth stem-and-loop structural body, the fourth stem-and-loop structural body comprises a fourth double strand region and a fourth single strand region on one strand, the fourth double strand region comprises a fourth stem portion derived from a complementary sequence of the S2 sequence and a sequence derived from a complementary sequence of the second template sequence, the sequence derived from a complementary sequence of the second template sequence is complementary to the fourth stem portion, the fourth single strand region forms a fourth loop portion, and the fourth single strand region, which forms the fourth loop portion, comprises a complementary sequence to the S1 sequence;
(a-2) preparing at least one from the group consisting of a first nucleic acid probe, a second nucleic acid probe, a third nucleic acid probe and a fourth nucleic acid probe, configured to detect the first replication strand and/or the second replication strand,
wherein the first nucleic acid probe comprises a sequence of at least a part of the first single strand region forming the first loop portion in the first replication strand,
the second nucleic acid probe comprises a sequence of at least a part of the second single strand region forming the second loop portion in the second replication strand,
the third nucleic acid probe comprises a sequence of at least a part of the third single strand region forming the third loop portion in the third replication strand, the sequence comprising a sequence of at least a part of the complementary sequence to the S3 sequence, and
the fourth nucleic acid probe comprises a sequence of at least a part of the fourth single strand region forming the fourth loop portion in the fourth replication strand, the sequence comprising a sequence of at least a part of the S1 sequence;
(b) amplifying the sample using the first primer and the second primer,
(c) reacting an amplification product obtained in (b) with the at least one from the group consisting of the first nucleic acid probe, the second nucleic acid probe, the third nucleic acid probe and the fourth nucleic acid probe,
(d) detecting presence/absence of a hybridization and/or measuring a quantity of the hybridization occurred due to the reaction in the (C), and
(e) determining presence/absence of the target nucleic acid in the sample and/or determining a quantity of target nucleic acid presence, based on a result in the (d).

8. The nucleic acid analysis method of claim 7, wherein the amplification is by a PCR amplification reaction.

9. The nucleic acid analysis method of claim 8, wherein the nucleic acid probe is immobilized on a substrate.

10. The nucleic acid analysis method of claim 9, wherein the detecting the hybridization and/or measuring the quantity of the hybridization between the nucleic acid probe immobilized on the substrate and the amplification product is carried out using an intercalating agent which indicates an electrochemical activity.

11. The nucleic acid analysis method of claim 10, wherein the sample comprises a gene and an object of the analysis is the gene.

12. An assay kit used for the nucleic acid analysis method of claim 7, the assay kit comprising:
a primer set and a nucleic acid probe,
wherein a first template nucleic acid to be amplified with the primer set comprises a first sequence located on a 3' terminal, a first template sequence adjacent to a 5' side of the first sequence, a second sequence adjacent to a 5' side of the first template sequence and located on the 5' terminal, and a second template nucleic acid to be amplified with the primer set is a strand complementary to the first template nucleic acid, and comprises a third sequence, located on a 3' terminal thereof, a second template sequence adjacent to a 5' side of the third sequence, and a fourth sequence adjacent to a 5' side of the second template sequence and located on the 5' terminal,
the primer set satisfies the following (1) and/or (2) :
(1) the first primer comprises an S1 sequence and an S2 sequence, the S1 sequence being complementary to the first sequence, the S2 sequence joined to a 5' terminal side of the S1 sequence, the S2 sequence being a sequence, which is same as a sequence of at least a part of the first template sequence, located adjacent to or in a vicinity of a 5' side of the first sequence on the first template nucleic acid, and
the second primer comprises an S3 sequence complementary to the third sequence;
(2) the first primer comprises an S1 sequence and an S2 sequence, the S1 sequence being complementary to the first sequence, and the S2 sequence joined to a 5' side of the S1 sequence, the S2 sequence being a sequence, which is same as a sequence of at least a part of the first template sequence, located adjacent to or in a vicinity of a 5' side of the S1 sequence on the first template nucleic acid, and
the second primer comprises an S3 sequence and an S4 sequence, the S3 sequence being complementary to the third sequence, the S4 sequence joined to a 5' side of the S3 sequence, the S4 sequence being a sequence, which is same as a sequence of at least a part of the second template sequence, located adjacent to or in a vicinity of a 5' side of the third sequence on the second template nucleic acid;
the nucleic acid probe is at least one type selected from the group consisting of:
a nucleic acid probe comprising a sequence of at least a part of the S1 sequence;
a nucleic acid probe comprising a sequence of at least a part of the complementary sequence of the S1 sequence;
a nucleic acid probe comprising a sequence of at least a part of the S3 sequence; and
a nucleic acid probe comprising a sequence of at least a part of the complementary sequence of the S3 sequence.


SEQUENCE LISTING

<110>  Kabushiki Kaisha Toshiba

<120>  Method for analyzing of a nucleic acid

<130>  12S0763P

<160>  19

<170>  PatentIn version 3.4

<210>  1
<211>  1507
<212>  DNA
<213>  Bacillus subtilis

<400>  1
aggacgaacg ctggcggcgt gcctaataca tgcaagtcga gcggacagat gggagcttgc      60

tccctgatgt tagcggcgga cgggtgagta acacgtgggt aacctgcctg taagactggg     120

ataactccgg gaaaccgggg ctaataccgg atgcttgttt gaaccgcatg gttcaaacat     180

aaaaggtggc ttcggctacc acttacagat ggacccgcgg cgcattagct agttggtgag     240

gtaatggctc accaaggcaa cgatgcgtag ccgacctgag agggtgatcg gccacactgg     300

gactgagaca cggcccagac tcctacggga ggcagcagta gggaatcttc cgcaatggac     360

gaaagtctga cggagcaacg ccgcgtgagt gatgaaggtt ttcggatcgt aaagctctgt     420

tgttagggaa gaacaagtac cgttcgaata gggcggtacc ttgacggtac ctaaccagaa     480

agccacggct aactacgtgc cagcagccgc ggtaatacgt aggtggcaag cgttgtccgg     540

aattattggg cgtaaagggc tcgcaggcgg ttccttaagt ctgatgtgaa agcccccggc     600

tcaaccgggg agggtcattg gaaactgggg aacttgagtg cagaagagga gagtggaatt     660

ccacgtgtag cggtgaaatg cgtagagatg tggaggaaca ccagtggcga aggcgactct     720

ctggtctgta actgacgctg aggagcgaaa gcgtggggag cgaacaggat tagataccct     780

ggtagtccac gccgtaaacg atgagtgcta agtgttaggg ggtttccgcc ccttagtgct     840

gcagctaacg cattaagcac tccgcctggg gagtacggtc gcaagactga aactcaaagg     900

aattgacggg ggcccgcaca agcggtggag catgtggttt aattcgaagc aacgcgaaga     960

accttaccag gtcttgacat cctctgacaa tcctagagat aggacgtccc cttcgggggc    1020

agagtgacag gtggtgcatg gttgtcgtca gctcgtgtcg tgagatgttg ggttaagtcc    1080

cgcaacgagc gcaacccttg atcttagttg ccagcattca gttgggcact ctaaggtgac    1140

tgccggtgac aaaccggagg aaggtgggga tgacgtcaaa tcatcatgcc ccttatgacc    1200

tgggctacac acgtgctaca atggacagaa caaagggcag cgaaaccgcg aggttaagcc    1260

aatcccacaa atctgttctc agttcggatc gcagtctgca actcgactgc gtgaagctgg    1320

aatcgctagt aatcgcggat cagcatgccg cggtgaatac gttcccgggc cttgtacaca    1380

ccgcccgtca caccacgaga gtttgtaaca cccgaagtcg gtgaggtaac cttttaggag    1440

ccagccgccg aaggtgggac agatgattgg ggtgaagtcg taacaaggta gccgtatcgg 1500

aaggtgc 1507

<210> 2
<211> 23
<212> DNA
<213> artificial

<220>
<223> Primer

<400> 2
gggagcgaac aggattagat acc 23

<210> 3
<211> 19
<212> DNA
<213> artificial

<220>
<223> Primer

<400> 3
ctgacgacaa ccatgcacc 19

<210> 4
<211> 20
<212> DNA
<213> artificial

<220>
<223> Primer

<400> 4
gtaggtggca agcgttgtcc 20

<210> 5
<211> 20
<212> DNA
<213> artificial

<220>
<223> Primer

<400> 5
agcactaagg ggcggaaacc 20

<210> 6
<211> 47
<212> DNA
<213> artificial

<220>
<223> Primer

<400> 6
ccctaacact tagcactcat cgttgggagc gaacaggatt agatacc 47

<210>  7
<211>  43
<212>  DNA
<213>  artificial

<220>
<223>  Primer

<400>  7
gaaaccccct aacacttagc gggagcgaac aggattagat acc                43


<210>  8
<211>  43
<212>  DNA
<213>  artificial

<220>
<223>  Primer

<400>  8
agcactaagg ggcggaaacc gggagcgaac aggattagat acc                43


<210>  9
<211>  40
<212>  DNA
<213>  artificial

<220>
<223>  Primer

<400>  9
ggtagtccac gccgtaaacg agcactaagg ggcggaaacc                    40


<210>  10
<211>  42
<212>  DNA
<213>  artificial

<220>
<223>  Primer

<400>  10
attagatacc ctggtagtcc acagcactaa ggggcggaaa cc                 42


<210>  11
<211>  44
<212>  DNA
<213>  artificial

<220>
<223>  Primer

<400>  11
ggagcgaaca ggattagata ccctagcact aaggggcgga aacc               44


<210>  12
<211>  20
<212>  DNA
<213>  artificial

<220>
<223>  Primer

<400>  12
gcgtagagat gtggaggaac                                                    20


<210>  13
<211>  22
<212>  DNA
<213>  artificial

<220>
<223>  Primer

<400>  13
aggcagcagt agggaatctt cc                                                 22


<210>  14
<211>  23
<212>  DNA
<213>  artificial

<220>
<223>  Primer

<400>  14
cctgtaagac tgggataact ccg                                                23


<210>  15
<211>  40
<212>  DNA
<213>  artificial

<220>
<223>  Primer

<400>  15
gctcctcagc gtcagttaca gcgtagagat gtggaggaac                              40


<210>  16
<211>  45
<212>  DNA
<213>  artificial

<220>
<223>  Primer

<400>  16
gctttcacat cagacttaag gaaccgtagg tggcaagcgt tgtcc                        45


<210>  17
<211>  30
<212>  DNA
<213>  artificial

<220>
<223>  Probe

<400>  17

acttagcact catcgtttac ggcgtggact 30

<210> 18
<211> 40
<212> DNA
<213> artificial

<220>
<223> Probe

<400> 18
ctaacactta gcactcatcg tttacggcgt ggactaccag 40

<210> 19
<211> 30
<212> DNA
<213> artificial

<220>
<223> Probe

<400> 19
tgcttctaca cagtctcctg tacctgggca 30